# EUROPEAN PATENT SPECIFICATION

(11) **EP 3 792 359 B1**
(45) Date of publication and mention of the grant of the patent: **09.12.2026**
(21) Application number: 20203177.9
(22) Date of filing: 30.03.2012
(51) Int. Cl.: C12N 15/09

(54) **FUNCTIONAL NUCLEIC ACID MOLECULE AND USE THEREOF**
FUNKTIONELLES NUKLEINSÄUREMOLEKÜL SOWIE VERWENDUNG DAVON
MOLÉCULE D'ACIDE NUCLÉIQUE FONCTIONNELLE ET SES APPLICATIONS

(30) Priority: 30.03.2011 US 201161469399 P
(43) Date of publication of application: 17.03.2021
(62) Document number(s) of the earlier application(s) in accordance with Art. 76 EPC: 12763652.0 / 2 691 522
(73) Proprietor: Harness Therapeutics Limited, Cambridge CB22 3AT (GB); International School For Advanced Studies, Trieste 34136 (IT)
(72) Inventor: CARNINCI, Piero, Yokohama-shi, Kanagawa 230-0045 (JP); FORREST, Alistair, Yokohama-shi, Kanagawa 230-0045 (JP); GUSTINCICH, Stefano, 34134 Trieste (IT); CARRIERI, Claudia, 00015 Rome (IT); ZUCCHELLI, Silvia, deceased (IT)
(74) Representative: Zaccaro, Elisabetta

(56) References cited:
- WO-A2-2009/083790
- KAWAJI HIDEYA ET AL: "The FANTOM web resource: from mammalian transcriptional landscape to its dynamic regulation", GENOME BIOLOGY, BIOMED CENTRAL LTD., LONDON, GB, vol. 10, no. 4, 19 April 2009 (2009-04-19), pages R40, XP021053588, ISSN: 1465-6906, DOI: 10.1186/GB-2009-10-4-R40
- T. SASAKI ET AL: "Possible involvement of SINEs in mammalian-specific brain formation", PROCEEDINGS OF THE NATIONAL ACADEMY OF SCIENCES, vol. 105, no. 11, 18 March 2008 (2008-03-18), pages 4220 - 4225, XP055142579, ISSN: 0027-8424, DOI: 10.1073/pnas.0709398105
- CLAUDIA CARRIERI ET AL: "Long non-coding antisense RNA controls Uchl1 translation through an embedded SINEB2 repeat", NATURE, vol. 491, no. 7424, 14 October 2012 (2012-10-14), pages 454 - 457, XP055142699, ISSN: 0028-0836, DOI: 10.1038/nature11508
- WEN-YU SU ET AL: "Natural antisense transcripts regulate gene expression in an epigenetic manner", BIOCHEMICAL AND BIOPHYSICAL RESEARCH COMMUNICATIONS, vol. 396, no. 2, 1 May 2010 (2010-05-01), pages 177 - 181, XP055142303, ISSN: 0006-291X, DOI: 10.1016/j.bbrc.2010.04.147

## Description

### Technical Field

The present invention relates to (a) a functional nucleic acid molecule having a function of increasing the protein synthesis efficiency, and (b) the functional nucleic acid molecule for use as defined in the claims.

### Background Art

There are various types of functional nucleic acid molecules, typified by relatively long functional RNA molecules such as antisense RNA, for example. Other typical examples of the functional nucleic acid molecules are relatively short functional RNA molecules such as shRNA (short hairpin RNA), siRNA (small interfering RNA), and miRNA (micro interfering RNA). These functional RNA molecules are generally known to contribute to down-regulation of gene expression. Various examples have been broadly reviewed as "interfering RNA" like in (Non Patent Literature 1).

An antisense RNA technique is excellent in target specificity. However, this technique has a disadvantage that an antiviral response in a cell is activated. In contrast, the technique using the relatively short functional RNA molecules such as shRNA does not activate the antiviral response practically. Instead, the relative short functional RNA molecules have the possibility of causing the off-target effects because it is difficult for short sequence length to keep high specificity to the target sequences. Thus, the relatively short functional RNA molecules tend to exhibit poor target specificity as compared with the antisense RNA technique.

Patent Literature 1 discloses a functional nucleic acid molecule (DNA molecule) comprising: a pol III type III promoter; a sequence identical or complementary to a target sequence that performs downregulation; and a 7SL small-RNA derived sequence (more specifically, a fragment of an Alu derived sequence) including at least binding domains to srp9 and srp14 proteins. The srp9 and srp14 proteins are members of a family of proteins that bind to a 7SL RNA in gene transcription to form a 7SL RNA complex.

It is described that the functional nucleic acid molecule disclosed in Patent Literature 1 is used as a gene expression downregulation technique in which an RNA molecule transcribed from the functional nucleic acid molecule practically causes no antiviral response activation and has excellent target specificity.

Alu is classified into one group of SINEs (Short Interspersed Elements). Note that the Alu derived sequence of the functional nucleic acid molecule disclosed in Patent Literature 1 is inserted in a particular orientation and considered to be involved in RNA stability.

On the other hand, it has been reported that some small RNA molecules can also enhance the level of transcription.

### Citation List

Patent Literature 1
   International Publication WO 2008/113773 A2 (Publication Date: September 25, 2008)
Non Patent Literature 1
   He L, Hannon GJ. Nat Rev Genet. 2004 Jul;5(7):522-31. PMID: 15211354

### Summary of Invention

### Technical Problem

As widely reported in Patent Literature 1 and other documents, many types of functional nucleic acid molecules that down-regulate gene expression or the like, are well known. Although there have been some techniques for up-regulating gene expression by increasing the transcription efficiency (like in the case of Nature Chemical Biology 3, 166 - 173 (2007), B. Janowski et al), increasing of the transcription efficiency does not always increase the efficiency of protein synthesis in direct proportion because of the plateau effect. In this regard, the synthesis of translated protein may depend on many other factors, including the ability of a given RNA to interact efficiently with the ribosomes. Additionally, increasing transcription of the natural mRNA is not always possible in the cells or organisms. That is, any functional nucleic acid molecule that increases the protein synthesis efficiency directly has not been reported.

The present inventors consider that there are many conditions in which acting only on translation is desired. For instance, enhancement of translation of an animal protein for therapeutic purposes without interfering with its transcription may be highly desired because it does not require reprogramming of mRNA transcription in the nucleus.

The present invention is accomplished in view of the above problem. An object of the present invention is to provide a functional nucleic acid molecule having a function of increasing the protein synthesis efficiency, and a use of the functional nucleic acid molecule.

The functional nucleic acid molecules, the functional nucleic acid molecules for use, and the methods for the manufacture and use of the functional nucleic acid molecules of the invention are defined in the claims.

### Solution to Problem

The present inventors' current knowledge suggested that translation of protein may be regulated by factors including the structure of the regions of the mRNAs that is placed upstream the translated fraction of the mRNAs. This region is known as 5' UTR (5' untranslated region). For the purpose of this invention, the 5' UTR can be a natural one (found in natural RNAs, from the transcription starting site to the protein initiation codon), or can be an artificial sequence, such as the sequence present in a cloning vector or any other recombinant sequence.

The present inventors studied diligently to achieve the above object. Consequently, through analyses of functions of RNA molecules known as non-coding RNAs, the inventors found a surprising fact that a particular structure of such an RNA molecule has a function of increasing the protein synthesis efficiency. This functional nucleic acid exhibited the effect against a specific targeted protein through antisense sequence to a target sequence. Based on the findings, the inventors have accomplished the present invention.

That is, a functional nucleic acid molecule according to the present invention comprises:
(a) a target determinant sequence comprising antisense sequence to a target sequence in the protein-encoding RNA for which protein synthesis efficiency is to be increased; and
(b) a regulatory sequence having an activity of increasing of the protein synthesis efficiency wherein the regulatory sequence comprises a SINE (Short Interspersed Element) B2 derived sequence with at least 60% similarity to the RNA which is encoded by a DNA consisting of SEQ ID NO: 1, 2 or 3.

In the functional nucleic acid molecule according to the present invention, the regulatory sequence comprises a SINE (Short Interspersed Element)-derived sequence. Specifically, SINE-derived sequence according to the invention is a tRNA derived SINE, SINE B2, also described herein but not claimed are SINE-derived sequences from ID element, MEN, 4.5S1, DIP-derived sequence, or sequences that comprise joining separate elements of these sequences or similar sequences. SINE-derived sequence may be a sequence which comprises substantially potential predicted structures formed by parts of the SINE sequences, for example.

In the functional nucleic acid molecule according to the present invention, the regulatory sequence may be selected from the group consisting of the following (1) through (5):
(1) an RNA, which is encoded by a DNA consisting of the nucleotide sequence shown in SEQ ID No:1 (SINE/B2 in AS Uchl1)
(2) an RNA, which is encoded by a DNA consisting of the nucleotide sequence shown in SEQ ID No:2 (SINE/B2 , 39nt spacer indicated as underline and SINE/Alu in AS Uchl1)
(3) an RNA, which is encoded by a DNA consisting of the nucleotide sequence shown in SEQ ID No:3 (SINE/B2 in AS Uxt)
(4) nucleic acids (i) which is at least 60 % similarity to the RNA, which is encoded by a DNA consisting of the nucleotide sequence shown in SEQ ID No: 1, 2 or 3 and (ii) which has a function of increasing the protein synthesis efficiency; and
(5) nucleic acids (i) , which is encoded by a DNA in which not less than 1 but not more than 200 nucleotides are deleted, substituted, added, and/or inserted in the nucleotide sequence shown in SEQ ID No: 1, 2 or 3 and (ii) which has a function of increasing the protein synthesis efficiency.

In the functional nucleic acid molecule according to the present invention, the target determinant sequence comprises an antisense sequence to a target sequence in the protein-encoding RNA for which protein synthesis efficiency is to be increased.

In the functional nucleic acid molecule according to the present invention, the target determinant sequence may be located between a 5'-terminal and the regulatory sequence in the functional nucleic acid molecule.

In the functional nucleic acid molecule according to the present invention, it is preferable that the antisense sequence in the target determinant sequence have a length more than 7 nucleotides but less than 250 nucleotides.

In the functional nucleic acid molecule according to the present invention, it is preferable that the antisense sequence in the target determinant sequence be at least 60% similarity to a target sequence in the protein-encoding RNA or to the plasmid sequence upstream of the ATG in the protein-encoding RNA for which protein synthesis efficiency is to be increased.

In the functional nucleic acid molecule according to the present invention, it is preferable that the direction of the SINE-derived sequence which is annotated as forward in the regulatory sequence is oriented in a reverse direction relative to the direction (forward direction as defined above), wherein SINE-derived sequence is oriented in the same direction of the consensus sequence of SINE. That is, the regulatory sequence of the functional nucleic acid molecule is oriented in a direct direction relative to the direction of translation and reverse orientation relative to the direction of transcription of the antisense nucleic acid molecule.

If the direction from 5' to 3' is defined as the forward direction, the SINE-derived sequence in this invention, wherein its 5' to 3'orientation accords with the SINE consensus sequence, is embedded in the reverse direction of the functional nucleic acid molecule in this invention.

In the functional nucleic acid molecule according to the present invention, the antisense sequence in the target determinant sequence may be designed to hybridize with a target sequence in the 5'-UTR of the protein-encoding RNA for which protein synthesis efficiency is to be increased. Alternatively, the target determinant sequence may be designed to hybridize with a target sequence in the coding region of the protein-encoding RNA for which protein synthesis efficiency is to be increased. In addition, the target determinant sequence may overlap to the sequence of plasmid upstream of the starting codon or including the starting codon in the target sequence of the protein encoding RNA. The functional nucleic acid molecule according to the present invention can be targeted to specific splicing variants at the 5- ends of the protein coding RNA or in other parts of the molecule.

The functional nucleic acid molecule according to the present invention can be appropriately designed and produced by a skilled person in the related art, as long as the functional nucleic acid molecule includes: a target determinant sequence comprising antisense sequence to a target sequence in the protein-encoding RNA for which protein synthesis efficiency is to be increased; and a regulatory sequence having an activity of increasing of the protein synthesis efficiency. Any modification during or post/synthesis can be applied to the functional nucleic acid molecule by a skilled person in the related art according to the know/how in RNA therapeutics or the like.

A DNA molecule according to the present invention encodes any one of the RNA molecules as aforementioned functional nucleic acid molecules.

An expression vector according to the present invention includes any one of the RNA molecules or the DNA molecule as aforementioned functional nucleic acid molecules.

A composition for increasing protein synthesis efficiency according to the present invention comprises any one of the aforementioned functional nucleic acid molecules and/or the aforementioned expression vector.

An *in vitro* method for increasing the protein synthesis efficiency according to the present invention comprises the step of allowing any one of the aforementioned functional nucleic acid molecules or the aforementioned expression vector to coexist with a protein-encoding RNA for which protein synthesis efficiency is to be increased. The protein-encoding RNA is hybridizable with the antisense in the target determinant sequence of the functional nucleic acid molecule.

In the *in vitro* protein synthesis efficiency-increasing method of the present invention, the method may comprise the step of transfecting (or transducing) into a cell any one of the aforementioned functional nucleic acid molecules or the aforementioned expression vector.

An *in vitro* protein synthesis method according to the present invention is a method for synthesizing a protein, comprising the step of increasing the protein synthesis efficiency by any one of the aforementioned protein synthesis efficiency-increasing methods.

Described herein but not claimed is a method for treating a disease, wherein the disease is caused by a quantitative decrease in a predetermined normal protein or haploinsufficiency, comprises the step of increasing the protein synthesis efficiency in a subject by any one of the aforementioned protein synthesis efficiency-increasing methods.

In the treatment method described herein, the subject may have a disease or a predisposition to the disease, wherein the disease is caused by a quantitative decrease in a predetermined normal protein or haploinsufficiency. Furthermore, the functional nucleic acid molecule may increase the efficiency of synthesizing of the predetermined normal protein.

Further, in case that the disease is caused by a quantitative decrease in a predetermined normal protein or haploinsufficiency, and caused by a quantitative increase of another protein including an abnormal protein like mutant protein, the treatment method disclosed herein can be used in combination with a conventional method for suppressing the expression of the another protein by siRNA, shRNA, or the like, and/or a conventional method for inactivating a function of a protein by use of an antibody, a low-molecular-weight compound, or the like, as appropriate.

Further, in the treatment method according to the present disclosure, the disease may be a neurodegenerative disease or cancer, for example.

In one aspect, there is provided the functional nucleic acid molecule of the claimed invention, the DNA molecule of the claimed invention, or the expression vector of the claimed invention, for use in medicine.

In another aspect, there is provided the functional nucleic acid molecule of the claimed invention, the DNA molecule of the claimed invention, or the expression vector of the claimed invention, for use in the treatment of a haploinsufficiency or a disease that is caused by a quantitative decrease in a protein.

### Advantageous Effects of Invention

According to the present invention, it is successfully possible to provide (a) a functional nucleic acid molecule having a function of increasing the protein synthesis efficiency as defined in the claims and (b) a use of the functional nucleic acid molecule as defined in the claims.

References to methods of treatment by therapy in this description are to be interpreted as references to the products (e.g., functional nucleic acids) of the present invention for use in those methods.

### Brief Description of Drawings

Fig.1

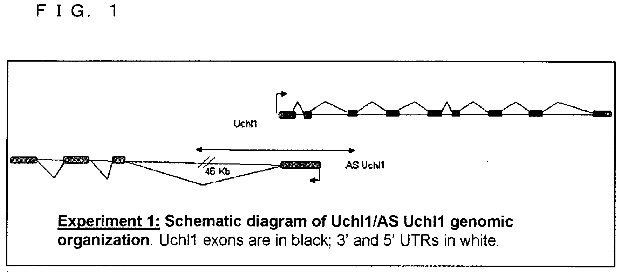

   Fig.1, related to the experiment 1, is a schematic diagram of Uchl1/AS Uchll genomic organization. Uchll exons are in black; 3' and 5' UTRs in white.
Fig.2

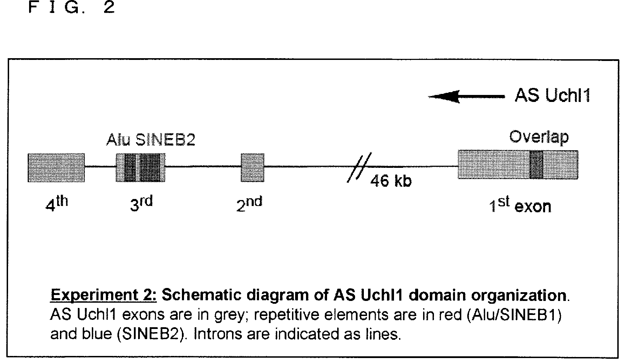

   Fig.2, related to the experiment2, is a schematic diagram of AS Uchl1 domain organization. AS Uchl1 exons are in grey; repetitive elements are in red (Alu/SINEB1) and blue (SINEB2). Introns are indicated as lines.
Fig.3

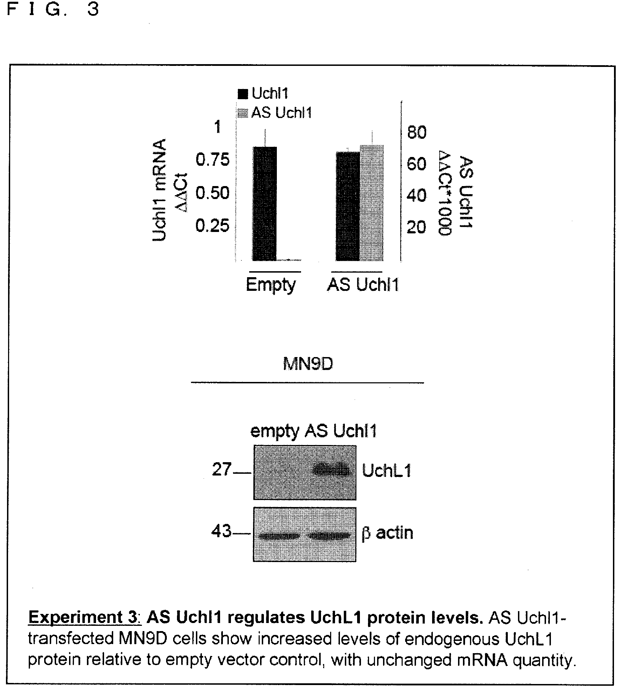

   Fig.3, related to the experiment3, is a view showing AS Uchl1 regulates UchL1 protein levels. AS Uchl1-transfected MN9D cells show increased levels of endogenous UchL1 protein relative to empty vector control, with unchanged mRNA quantity.
Fig.4

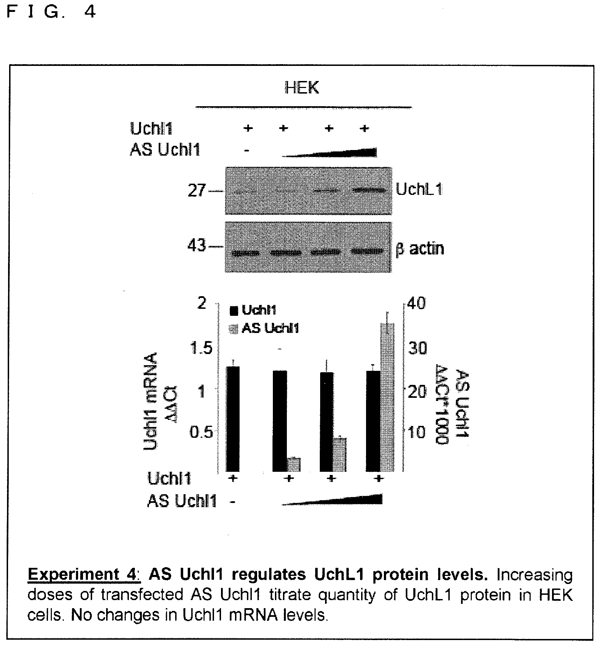

   Fig.4, related to the experiment4, is a view showing AS Uchl1 regulates UchL1 protein levels. Increasing doses of transfected AS Uchl1 titrate quantity of UchL1 protein in HEK cells. No changes in Uchl1 mRNA levels.
Fig.5

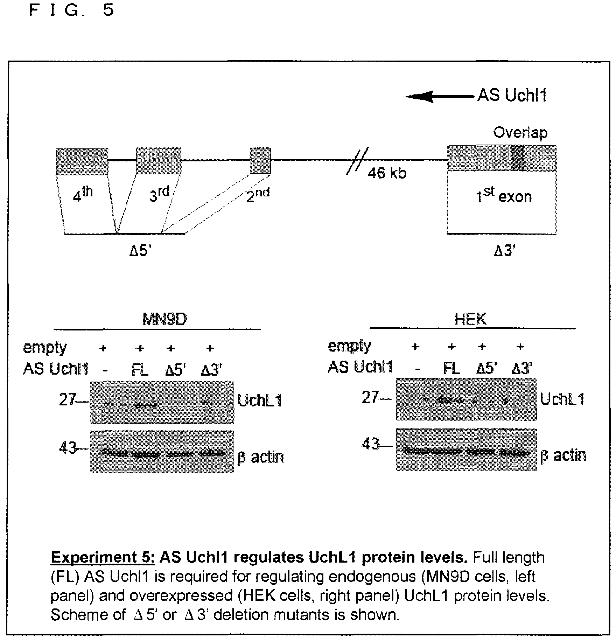

   Fig.5, related to the experiment5, is a view showing AS Uchl1 regulates UchL1 protein levels. Full length (FL) AS Uchl1 is required for regulating endogenous (MN9D cells, left panel) and overexpressed (HEK cells, right panel) UchL1 protein levels. Scheme of Δ5' or Δ3' deletion mutants is shown.
Fig.6

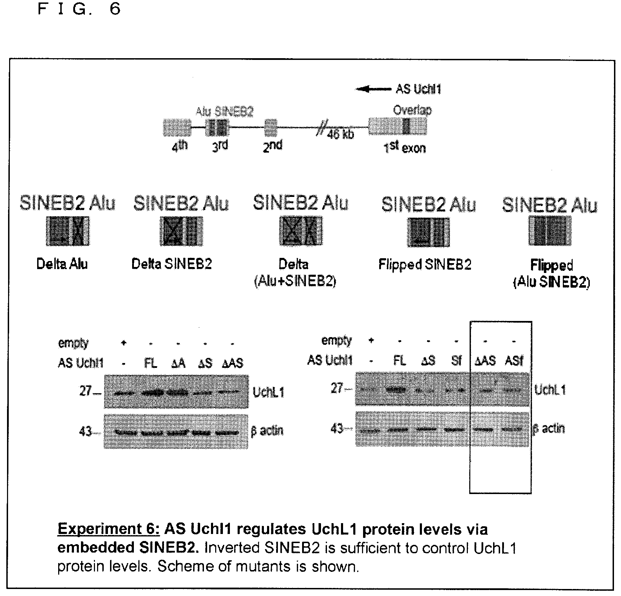

   Fig.6, related to the experiment6, is a view showing AS Uchl1 regulates UchL1 protein levels via embedded SINEB2. Inverted SINEB2 is sufficient to control UchL1 protein levels. Scheme of mutants is shown.
Fig.7

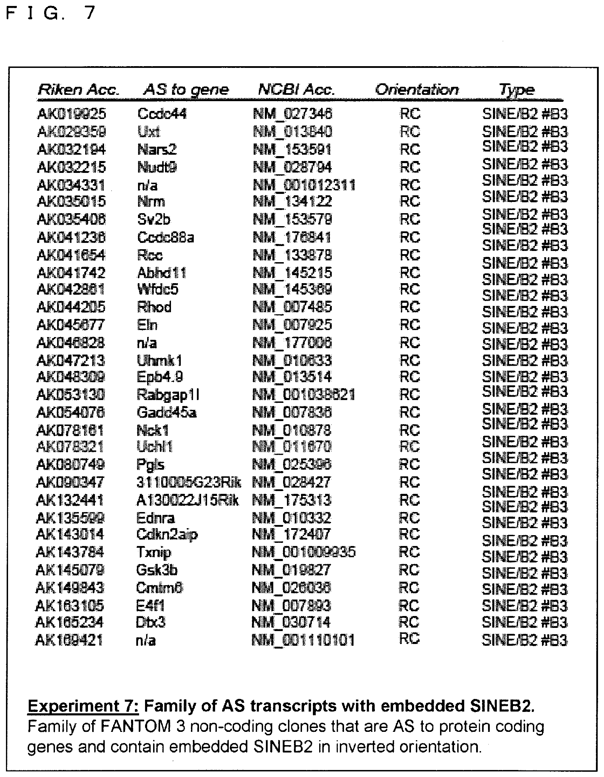

   Fig.7, related to the experiment7, is a view showing Family of AS transcripts with embedded SINEB2. Family of FANTOM 3 non-coding clones that are AS to protein coding genes and contain embedded SINEB2 in inverted orientation.
Fig.8

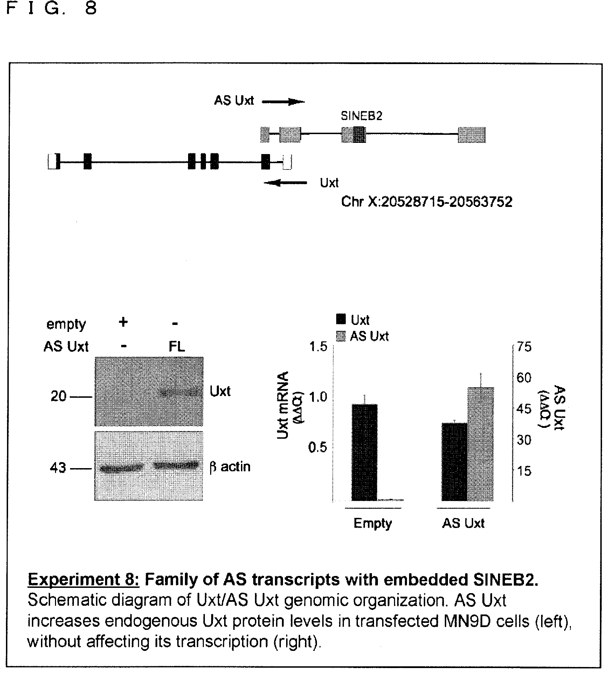

   Fig.8, related to the experiment8, is a view showing Family of AS transcripts with embedded SINEB2. Schematic diagram of Uxt/AS Uxt genomic organization. AS Uxt increases endogenous Uxt protein levels in transfected MN9D cells (left), without affecting its transcription (right).
Fig.9

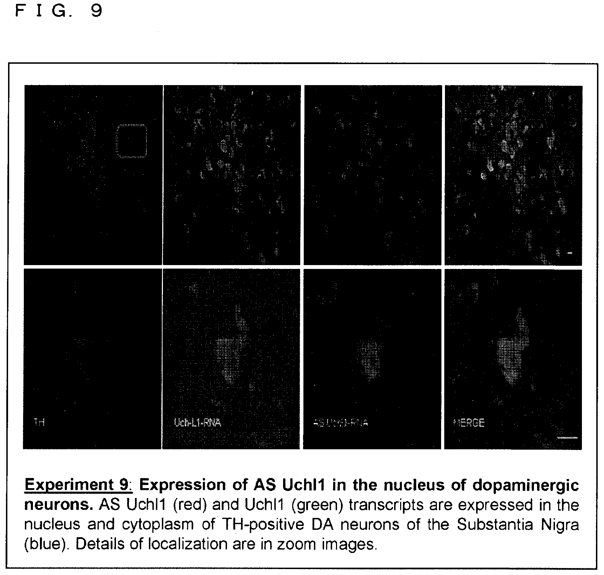

   Fig.9, related to the experiment 9, is a view showing Expression of AS Uchll in the nucleus of dopaminergic neurons. AS Uchll (red) and Uchll (green) transcripts are expressed in the nucleus and cytoplasm of TH-positive DA neurons of the Substantia Nigra (blue). Details of localization are in zoom images.
Fig. 10

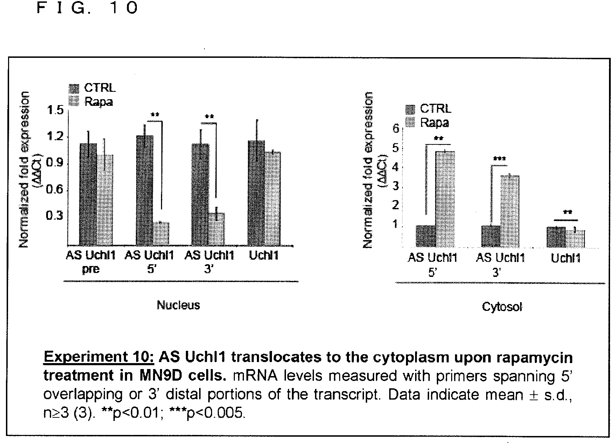

   Fig. 10, related to the experiment 10, is a view showing AS Uchll translocates to the cytoplasm upon rapamycin treatment in MN9D cells. mRNA levels measured with primers spanning 5' overlapping or 3' distal portions of the transcript. Data indicate mean ± s.d., n≥3 (3). **p<0.01; ***p<0.005.
Fig. 11

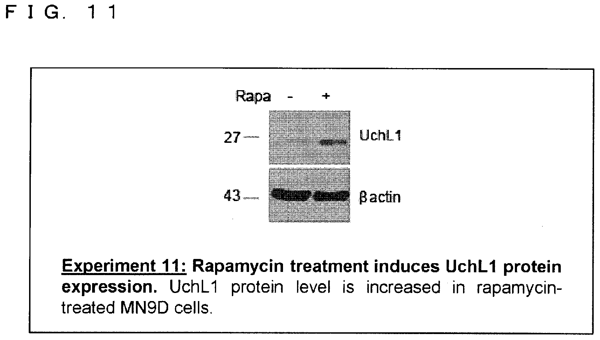

   Fig. 11, related to the experiment 11, is a view showing Rapamycin treatment induces UchL1 protein expression. UchL1 protein level is increased in rapamycin-treated MN9D cells.
Fig.12

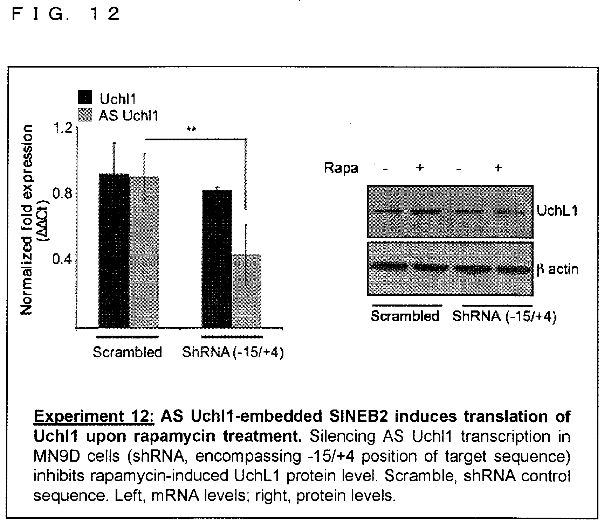

   Fig. 12, related to the experiment 12, is a view showing AS Uchl1-embedded SINEB2 induces translation of Uchll upon rapamycin treatment. Silencing AS Uchll transcription in MN9D cells (shRNA, encompassing -15/+4 position of target sequence) inhibits rapamycin-induced UchL1 protein level. Scramble, shRNA regulatory sequence. Left, mRNA levels; right, protein levels.
Fig.13

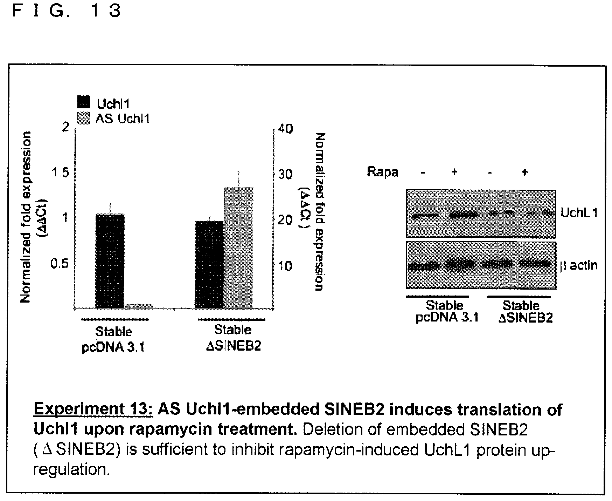

   Fig. 13, related to the experiment 13, is a view showing AS Uchi1-embedded SINEB2 induces translation of Uchll upon rapamycin treatment. Deletion of embedded SINEB2 (ΔSINEB2) is sufficient to inhibit rapamycin-induced UchL1 protein up-regulation.
Fig.14

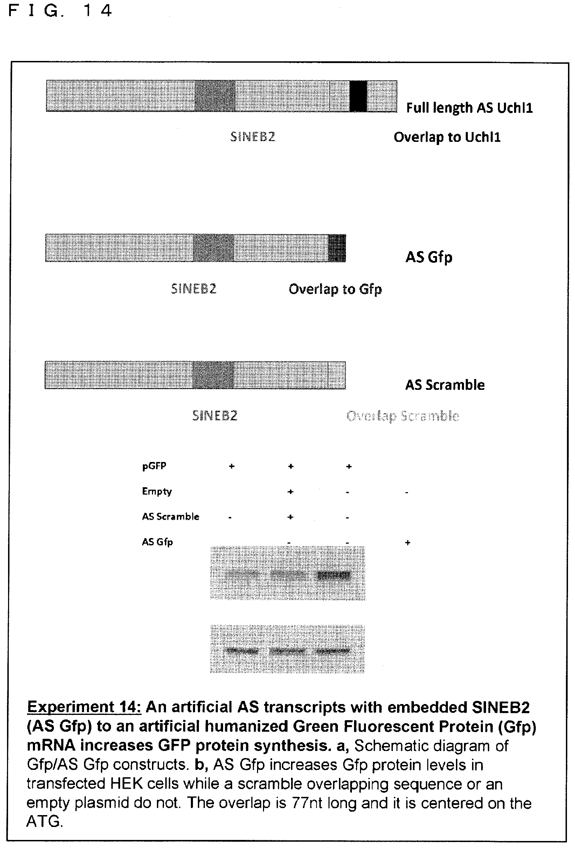

   Fig. 14, related to the experiment 14, is a view showing an artificial AS transcript with embedded SINEB2 (AS Gfp) to an artificial humanized enhanced Green Fluorescent Protein (Gfp) mRNA increases GFP protein synthesis: (a) of Fig. 14 shows Schematic diagram of Gfp/AS Gfp constructs, (b) of Fig,. 14 shows how AS Gfp increases Gfp protein levels in transfected HEK cells while a scramble overlapping sequence or an empty plasmid does not increases Gfp protein levels in transfected HEK cells. The overlap is 72nt long and it is centered on the ATG.
Fig.15

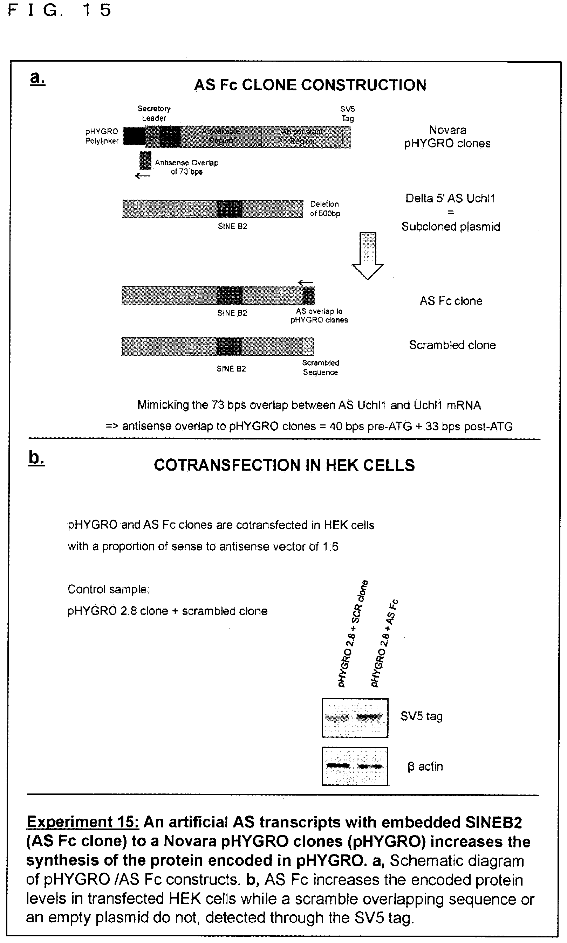

   Fig. 15, related to the experiment 15, is a view showing an artificial AS transcript with embedded SINEB2 (AS Fc clone) to an artificial recombinant antibody in pHYGRO (pHYGRO clones) increases the synthesis of the protein encoded in pHYGRO. a, Schematic diagram of pHYGRO /AS Fc constructs. b, AS Fc increases the encoded protein levels in transfected HEK cells while a scramble overlapping sequence or an empty plasmid do not, detected through the SV5 tag.
Fig.16

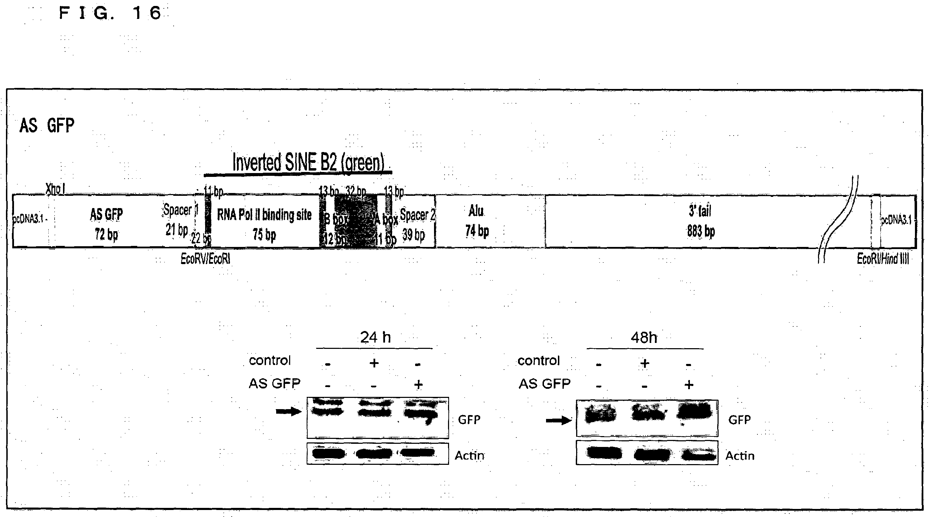

   Fig. 16, related to the experiment 16, is a view showing how an artificial AS transcript with embedded SINEB2 to an artificial Green Fluorescent Protein (EGFP) mRNA increases EGFP protein synthesis.
Fig. 17

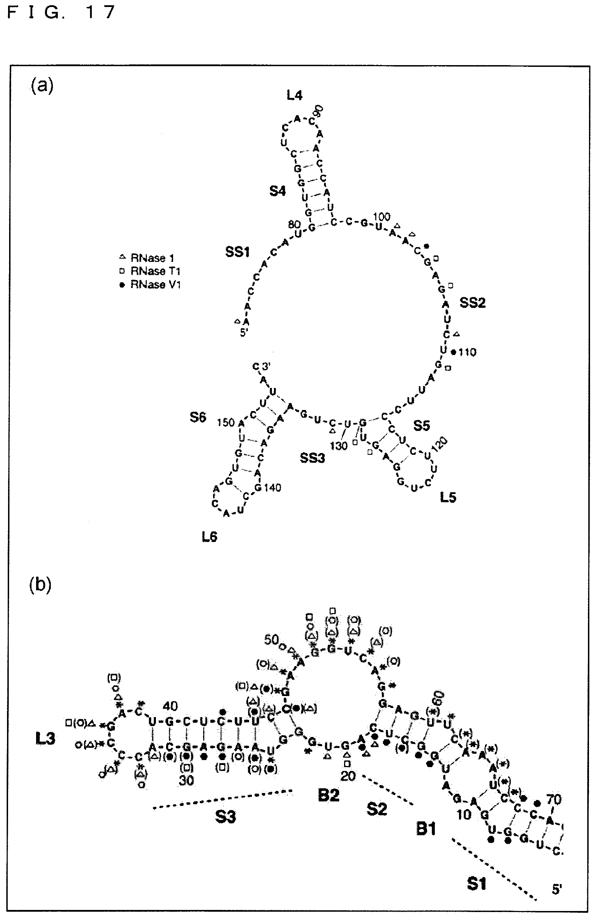

   Fig. 17 is a view showing the examples of the potential predicted structures of SINE B2-derived sequence.

### Description of Embodiments

The following describes an embodiment of the present invention, more specifically.

### [1. Functional RNA Molecule

### (Constitution of Functional nucleic acid Molecule)

A functional nucleic acid molecule according to the present invention has a feature that it comprises: a target determinant sequence comprising an antisense sequence to a target sequence in the protein-encoding RNA for which protein synthesis efficiency is to be increased; and a regulatory sequence having an activity of increasing of the protein synthesis efficiency wherein the regulatory sequence comprises a SINE (Short Interspersed Element) B2 derived sequence with at least 60% similarity to the RNA which is encoded by a DNA consisting of SEQ ID NO: 1, 2 or 3. The functional nucleic acid molecule according to the present invention can be appropriately designed and produced by a skilled person in the related art based on the description herein.

The protein synthesis efficiency is increased according to the present invention. In one embodiment, the protein synthesis efficiency is increased as a result of increase in the translation efficiency, preferably, without changing the transcription efficiency substantially. Thus, the protein synthesis efficiency may be increased by increasing the translation efficiency. In another embodiment, both transcription and translation can be increased by independent means; obviously, it may be the case that an RNA may result lower expression level, and yet the protein results higher expression level.

### (Protein-Encoding RNA for Which Protein Synthesis Efficiency is to be Increased)

The protein-encoding RNA for which protein synthesis efficiency is to be increased by the functional nucleic acid molecule according to the present invention is not especially limited in regard to its sequence, origin, and the like, provided that the RNA comprises a translation domain (coding region) having a 5'-terminal start codon and a 3'-terminal stop codon. Specifically, the protein-encoding RNA for which protein synthesis efficiency (the target RNA) is to be increased by the functional nucleic acid molecule according to the present invention may further have a 5'-cap structure, a 5' untranslated region (5'-UTR) and/or a 3' untranslated region. These regions may be derived from endogenous sequence in a cell or artificially synthesized sequence. The ORFeome, in which Open Reading Frames (coding sequences) of genes are placed in expression vectors, is one of the examples.

Further, it is preferable that the 3' untranslated region includes, at its 3' terminal, a sequence (poly-A addition signal) so that a poly-A sequence can be added. The poly-A addition signal may be, for example, a nucleotide sequence consisting of AAUAAA, a SV40 early poly-A signal having two sequences of AAUAAA, a sequence in which SV40 early poly-A signals are aligned in a tandem manner, or the like. The poly-A addition signal is not limited to them. As examples, various alternative polyadenylation sites have been described in the literature, and some mRNA do not even carry conventional polyadenylation signals (Carninci et al, Genome Res. 2003 Jun;13(6B):1273-89. PMID: 12819125).

The protein-encoding RNA for which protein synthesis efficiency is to be increased according to the present invention may have a poly-A sequence at its 3' terminal. An RNA having a poly-A sequence at its 3' terminal has excellent protein synthesis efficiency from a translation domain and excellent stability of the RNA itself. Such an RNA may be, for example, any one of RNAs shown in Uchll, Uxt, GFP or a homologue of any one of these RNAs, although such an RNA is not limited to them.
Mus musculus ubiquitin carboxy-terminal hydrolase L1 (Uchl1) RefSeq: NM_011670.2
The Uchll DNA sequence which encodes the Uchll RNA is shown in SEQ ID NO.4.
Mus musculus ubiquitously expressed transcript (Uxt) RefSeq: NM_013840.3
The Uxt DNA sequence which encodes the Uxt RNA is shown in SEQ ID NO.5.

### GFP(Sequence from pEGFP vector)

The GFP DNA sequence which encodes the GFP RNA sequence is shown in SEQ ID NO.6.

The protein-encoding RNA for which protein synthesis efficiency is to be increased may be endogenous RNA of biological origin (for example, mRNA) or artificially synthesized. Further, mRNA derived from eucaryotes encompass mature mRNA that has been subjected to what is called processing, and precursor mRNA that has not been subjected to processing.

### (Target Determinant Sequence)

The target determinant sequence is a sequence that comprises an antisense sequence to a target sequence in the protein-encoding RNA for which protein synthesis efficiency is to be increased. A target sequence is arbitrarily selected from a partial sequence of protein-encoding RNA for which protein synthesis efficiency is to be increased in the present invention. A target sequence may derive from the RNA sequence transcribed from the plasmid DNA in which the protein-encoding cDNA is inserted and around the first 5'-terminal start codon. A length of the antisense sequence is not especially limited. However, from the viewpoint of increasing specificity for a target RNA in a system including different RNAs, the antisense sequence may have a length of preferably more than 7 nucleotides, more preferably 10, more preferably 15. Furthermore, the antisense sequence may have a length of preferably less than 250 nucleotides, more preferably 200, more preferably 150, more preferably 100, more preferably 90, more preferably 80, more preferably 77 nucleotides, more preferably 70 nucleotides, more preferably 60 nucleotides, more preferably 50 nucleotides, more preferably 40 nucleotides, more preferably 30 nucleotides.

In one embodiment, more than one different antisense sequences can be included in the target determinant sequence. These multiple antisense sequences can be applied to targeting of multiple proteins, for example. Alternatively, the multiple antisense sequences can be applied to improving the specificity to a protein-encoding RNA wherein the multiple antisense sequences are hybridizable with the same protein-encoding RNA. In one embodiment, the target determinant sequence of the present invention may contain mismatches against the target RNA on purpose to prevent the reaction of gamma interferon that may take place in the cells in presented of long double strand nucleic acids molecule, like long double strand RNAs.

Further, from the viewpoint of increasing specificity between the functional nucleic acid molecule of the present invention and the target RNA, the antisense sequence in a target determinant sequence is designed to be preferably at least 60% similarity, more preferably at least 65% similarity, more preferably at least 70% similarity, more preferably at least 75% similarity, more preferably at least 80% similarity, more preferably at least 85% similarity, more preferably at least 90% similarity, more preferably at least 95% similarity to a corresponding target sequence in the protein-encoding RNA for which protein synthesis efficiency is to be increased, as long as the antisense sequence can hybridize to the target sequence in the protein-encoding RNA and/or to the plasmid-derived RNA that contains the target sequence. It is specifically preferable that the antisense sequence in the target determinant sequence be designed to be thoroughly identical with the corresponding sequence of the target sequence.

Further, an antisense sequence in a target determinant sequence can be designed to hybridize with a 5'-UTR of the protein-encoding RNA for which protein synthesis efficiency is to be increased. This design can be applied to the synthesizing full length encoded-protein. 5'-UTR may be derived from endogenous sequence in a cell or artificial sequence. The antisense sequence in the target determinant sequence may be designed to be hybridizable with other regions, except the 5'-UTR, of the target RNA, such as a coding region of the target RNA. For example, the antisense sequence in the target determinant sequence can be designed to be hybridizable with a given part of the coding region of the target RNA. This design is useful for a dystrophin gene or the like in which a protein to be encoded by an RNA is very large and has a domain that exhibits bioactivity by itself.

Additionally, the antisense sequence in the target determinant sequence can be designed to hybridize both the 5' UTR and a part of the other functional part of the sequence, like the coding sequence or the 3' UTRs of the protein coding mRNAs.

### (Regulatory sequence)

In the functional nucleic acid molecule according to the present invention, the regulatory sequence has an activity of increasing of the protein synthesis efficiency.

The regulatory sequence comprises a SINE-derived sequence as defined in the claims. Specifically, SINE-derived sequence is a tRNA-derived SINE,

SINE-B2. Other sequences described herein but not claimed are ID element, MEN, 4.5S1, DIP-derived sequence and 7SL-RNA-derived sequence such as Alu.

In one embodiment, multiple SINE-derived sequences can be included in the regulatory sequence. These multiple SINE-derived sequences may be with combination of different sequences, for example a combination of SINE B2-derived sequence and Alu-derived sequence or a combination of different SINE B2-derived sequence.

The SINE-derived sequence indicates a sequence entirely or partially identical with or similar to the consensus sequence of each species of SINE. For example, the SINE-B2-derived sequence indicates a sequence entirely or partially identical with or similar to the consensus sequence of a SINE B2. Even a truncated SINE derived sequence against the consensus sequence of SINE can be used as the SINE derived sequence in the present invention as long as a function of increasing the protein synthesize efficiency is kept. SINE-derived sequence may be a sequence which comprises substantially potential predicted structures formed by parts of the SINE sequences, for example. The examples of the potential predicted structures of SINE B2-derived sequence are as shown in (a) and (b) of Fig.16.

The similar sequence indicates a sequence that is at least 60% similarity, more preferably at least 65% similarity, more preferably at least 70% similarity, more preferably at least 75% similarity, more preferably at least 80% similarity, more preferably at least 85% similarity, more preferably at least 90% similarity, more preferably at least 95% similarity to the consensus sequence of the SINE.

The sequence of a SINE may deviate from these conservative consensus described above. For instance, the analysis of the consensus similarity between the SINE B2 sequences of three sequences alone, the SINE B2 fraction of SEQ ID NO:1, SEQ ID NO:3, and another SINE B2 randomly taken from the literature (reference: Espinosa et al, http://rnajournal.cship.org/content/13/4/583.full), clearly indicates that the SINE B2 fractions alone can share as little as 9 bases out of 36, with only 25% of similarity.

Yet, despite diverging, these are still recognizable as a SINE B2 element using programs like RepeatMask as published (Bioinformatics. 2000 Nov;16(11):1040-1.MaskerAid: a performance enhancement to RepeatMasker.Bedell JA, Korf I, Gish W.).

In one instance, the length can be limited to the shortest sequences with similarity to SINE elements, which are capable to cause increased the efficiency of protein synthesis.

In one instance, it is possible to synthesize artificial nucleic acid sequences that have partial similarity to the SINE elements described in the present invention and act to increase the level of synthesized protein.

Further, the functional nucleic acid molecule according to the present invention may include a plurality of regulatory sequences aligned in a tandem manner, for the purpose of further promoting the protein synthesis efficiency.

In the description of the present invention, "SINE" broadly indicates, among non-LTR (long terminal repeat) retrotransposon, an interspersed repetitive sequence (a) which encodes a protein having neither reverse-transcription activity nor endonuclease activity or the like and (b) whose complete or incomplete copy sequences exist abundantly in genomes of living organisms. That is, SINE is a DNA sequence that is inserted into a genome through the reverse transcription from RNA to cDNA, depending on other host factors in these processes. A length of the SINE is not especially limited, but generally, in a range of not less than 20 bp, preferably not less than 30bp, more preferably not less than 50bp, more preferably not less than 50bp, but not more than 700 bp, preferably not more than 600 bp, more preferably not more than 500 bp, more preferably not more than 400 bp. Further, the origin of SINE is not limited, but is generally derived from tRNA and has a sequence having a sequence corresponding to the tRNA on its 5'-terminal side. Further, the SINE may be a 7SL RNA-derived sequence such as Alu, and a 5S rRNA-derived sequence such as SINE 3. In regard to the SINE 3, the document by Kapitonov et al (Vladimir V. Kapitonov and Jerzy Jurka: Molecular Biology AND Evolution 20(5): p694-702, 2003) and the like document can be referred to.

The regulatory sequence may be, for example, selected from the following (1) to (5)
(1) an RNA, which is encoded by a DNA consisting of the nucleotide sequence shown in SEQ ID No:1
(2) an RNA, which is encoded by a DNA consisting of the nucleotide sequence shown in SEQ ID No:2
(3) an RNA, which is encoded by a DNA consisting of the nucleotide sequence shown in SEQ ID No:3
(4) nucleic acids (i) which is at least 60 % similarity to the RNA, which is encoded by a DNA consisting of the nucleotide sequence shown in SEQ ID No: 1, 2 or 3 and (ii) which has a function of increasing the protein synthesis efficiency; and
(5) nucleic acids (i) , which is encoded by an DNA in which not less than 1 but not more than 200 nucleotides are deleted, substituted, added, and/or inserted in the nucleotide sequence shown in SEQ ID No: 1, 2 or 3 and (ii) which has a function of increasing the protein synthesis efficiency.

The number of nucleotides to be deleted, substituted, added, and/or inserted is preferably not less than 1 but not more than 175, more preferably not less than 1 but not more than 150, more preferably not less than 1 but not more than 125, more preferably not less than 1 but not more than 100, more preferably not less than 1 but not more than 75, more preferably not less than 1 but not more than 50, more preferably not less than 1 but not more than 30, more preferably not less than 1 but not more than 20.

### (Positional Relationship between Target Determinant Sequence and Regulatory sequence)

In the present invention, a direction (a sense-strand direction) along which a target protein is translated is defined as "forward direction", and the direction opposite to the forward direction is defined as "reverse direction". In the functional nucleic acid molecule according to the present invention, a positional relationship between the target determinant sequence and the regulatory sequence is not especially limited. However, it is preferable that the target determinant sequence be located closer to a forward-direction side in the functional nucleic acid molecule than the regulatory sequence. The target determinant sequence may be directly linked to the regulatory sequence. Alternatively, a linker sequence and/or the like sequence for connecting the target determinant sequence and the regulatory sequence may be inserted there between.

In the functional nucleic acid molecule according to the present invention, it is preferable that the direction of the SINE-derived sequence which is annotated as forward in the regulatory sequence is oriented in a reverse direction relative to the direction (forward direction as defined above), wherein SINE-derived sequence is oriented in the same direction of the consensus sequence of SINE. That is, the regulatory sequence of the functional nucleic acid molecule is oriented in a forward direction relative to the direction of translation.

If the direction from 5' to 3' is defined as the forward direction, the SINE-derived sequence in this invention, wherein its 5' to 3'orientation accords with the SINE consensus sequence, is embedded in the reverse direction of the functional nucleic acid molecule in this invention. For example, in case of one of SINE B2-derived sequence, Abox site of SINE B2-derived sequence is located on the 3' side of the functional nucleic acid molecule compared to Bbox site of SINE B2-derived sequence.

### (Production of Functional nucleic acid Molecule)

An *in vitro* method according to the present invention for producing a functional nucleic acid molecule comprises the step of preparing the aforementioned RNA molecule of the invention. The functional nucleic acid molecules may be prepared by a well-known nucleic acid biosynthesis method, or such a method that (i) a DNA molecule encoding the functional RNA molecule is produced and (ii) the DNA molecule is transcribed into the functional RNA molecule, for example. A size of the functional nucleic acid molecule is not especially limited, but the functional nucleic acid molecule has a size of preferably not more than 2000 nucleotides, more preferably not more than 250 nucleotides, for example, from the viewpoint of producing the functional nucleic acid molecule by the nucleic acid biosynthesis method.

### [2. DNA Molecule, Expression Vector, Composition for Increasing Protein Synthesis Efficiency]

A DNA molecule according to the present invention encodes any one of the aforementioned functional nucleic acid molecules according to the present invention. Further, an expression vector according to the present invention is an RNA vector comprising any one of the aforementioned functional RNA molecules of the present invention or a DNA vector comprising the DNA molecule according to the present invention. Further, a composition for increasing protein synthesis efficiency according to the present invention comprises any one of the aforementioned functional nucleic acid molecules or the aforementioned expression vector.

In one embodiment, the composition according to the present invention may comprise a translation agent based on in vitro system like reticulocyte extract to produce protein in vitro; in mammalian cells expressing a protein for industrial use, for research purpose, or for any other screening, for example.

The backbone of the expression vector according to the present invention is not especially limited to any particular type, and may be appropriately selected from a plasmid vector. The plasmid vector may be a mammalian, yeast, insect expression vector, a virus vector (for example a lentiviral or retroviral expression vector, adenovirus or adeno-associated virus vectors), a phage vector, a cosmid vector, and the like, depending on types of host cells to be used and the purpose of use. For example, in a case where the present invention for use for gene treatment of mammals including a human, the present invention may be prepared in a form of a virus vector, such as an adenovirus or adeno-associated vector or a lentivirus vector.

Alternatively, the expression vector for use may ultimately be integrated in the genome of the expressing cells or organism to be targeted.

### [3. Method for Increasing Protein Synthesis Efficiency]

### (Method for Increasing Protein Synthesis Efficiency)

An *in vitro* method for increasing protein synthesis efficiency according to the present invention comprises the step of allowing a functional nucleic acid molecule according to the present invention or the aforementioned expression vector to coexist with a protein-encoding RNA for which protein synthesis efficiency is to be increased. This step can be carried out in vitro using, for example, cell-free protein synthesis system. In the present invention, "in vivo" means a system of using either cell culture or whole animal specifically, and "in vitro" means a system using cell-free assay specifically. In a case where the step is carried out in vivo, the functional nucleic acid molecule or the aforementioned expression vector may be allowed to coexist, in an isolated cell or tissue, with a protein-encoding RNA for which protein synthesis efficiency is to be increased. Alternatively, the functional nucleic acid molecule or the aforementioned expression vector may be allowed to coexist, in a living organism, with an RNA for which protein synthesis efficiency is to be increased.

The *in vitro* method for increasing protein synthesis efficiency according to the present invention may comprise transfecting into a cell an aforementioned expression vector encoding the functional nucleic acid molecule or the functional nucleic acid molecule itself so as to allow the functional nucleic acid molecule to coexist with the protein-encoding RNA. The "cell" indicates an isolated cell. The RNA for which protein synthesis efficiency is to be increased may be derived from an endogenous sequence in a cell or an RNA encoding a protein synthesized artificially. The transfection (or gene induction) of a nucleic acid molecule into a cell may be carried out appropriately by conventional methods, for example, self-infection by a vector, a microinjection technique, a lipofection technique, an electroporation technique, a calcium phosphate method, transduction of a virus and the like. The vector may or may not be permanently integrated in the host genome.

The cells may be derived from any one of cells from any organism including animals and plants, or any one of cells selected from the established cell lines.

In the present invention, animals include vertebrate, preferably mammals including a human, but are not limited to these examples.

In the present invention, plants include both monocotyledons and dicotyledons. In one embodiment, the plants are crop plants (for example, cereals and pulses, maize, wheat, potatoes, tapioca, rice, sorghum, millet, cassava, barley, or pea), or other legumes. In another embodiment, the plants may be vegetables or ornamental plants. The plants of the invention may be: corn (Zea mays), canola (Brassica napus, Brassica rapa ssp.), flax (Linum usitatissimum), alfalfa (Medicago sativa), rice (Oryza sativa), rye (Secale cerale), sorghum (Sorghum bicolour, Sorghum vulgare), sunflower (Helianthus annus), wheat (Tritium aestivum), soybean (Glycine max), tobacco (Nicotiana tabacum), potato, (Solanum tuberosum), peanuts (Arachis hypogaea), cotton (Gossypium hirsutum), sweet potato (Lopmoea batatus), cassava (Manihot esculenta), coffee (Cofea spp.), coconut (Cocos nucifera), pineapple (Anana comosus), citris tree (Citrus spp.), cocoa (Theobroma cacao), tea (Camellia senensis), banana (Musa spp.), avocado (Persea americana), fig (Ficus casica), guava (Psidium guajava), mango, (Mangifer indica), olive (Olea europaea), papaya (Carica papaya), cashew (Anacardium occidentale), macadamia (Macadamia intergrifolia), almond (Prunus amygdalus), sugar beets (Beta vulgaris), oats, or barley, but are not limited to these examples.

In the present invention, the established cell lines including mammalian derived cell such as COS-1 (ATCC No.CRL 1650), COS-7 (ATCC CRL 1651 ), human embryonic kidney line 293 (ATCC NO.CRL 1573), PerC6 (Crucell), baby hamster kidney cell (BHK) (ATCC CRL.1632), BHK570 (ATCC NO: CRL 10314), Chinese hamster ovary cells CHO (e.g. CHO-K1 , ATCC NO: CCL 61 , DHFR minus CHO cell line such as DG44, particularly those CHO cell lines adapted for suspension culture, mouse Sertoli cell, monkey kidney cell, African green monkey kidney cell (ATCC CRL-1587), HeLa cell, SH-Y5Y cell, canine kidney cell (ATCC CCL 34), human lung cell (ATCC CCL 75), Hep G2 and myeloma or lymphoma cells e.g. NSO (see US 5,807,715), Sp2/0, YO, other animals derived cells such as Sf9 cell,DT40, but are not limited to these examples. Furthermore, the established cell lines can be hybridoma or a cell given a particular feature by gene transfer, nuclear transfer and/or treatment of chemical compound, for example a nuclear transfer embryonic stem cell or an iPS cell(WO2007/069666, JP-A 2010-273680, JP-A 2010-284088, JP-A 2011-50379, JP-A 2011-4674, etc), a neuronal cell differentiated from neural stem cell, iPS cell or the like, or neuronal cells derived from re-programmed fibroblasts or the like, but are not limited to these examples.

The *in vitro* protein synthesis efficiency increasing method according to the present invention optimally contributes to increase the efficiency of the translation from RNA. The increase of the protein synthesis efficiency indicates that the protein synthesis efficiency is increased as compared with a case where the functional nucleic acid molecule according to the present invention or aforementioned expression vector is not allowed to coexist with the target RNA in a system. How much the protein synthesis efficiency is to be increased is not limited especially. However, it is preferable that an amount of a protein to be synthesized by the protein synthesis efficiency-increasing method be at least 1.5 times, more preferably at least 2 times more than an amount of a protein to be produced in the case where the functional RNA molecule is not allowed to coexist with the target RNA in the system.

In a case where the functional nucleic acid molecule or aforementioned expression vector is allowed to coexist with the target RNA in the system, a quantitative ratio between the functional nucleic acid molecule (or the expression vector) and the target RNA is not especially limited. The quantitative ratio between them may be, for example, 1:1 to 1:10.

### (Method for synthesizing Protein)

The aforementioned *in vitro* protein synthesis efficiency-increasing method according to the present invention can be used as a protein synthesis method. That is, a protein synthesis method according to the present invention is a method for producing a target protein, comprising the step of increasing the protein synthesis efficiency by any one of the aforementioned protein synthesis efficiency-increasing methods. It is preferable that the protein synthesis method allow for efficient synthesis of the target protein through the increasing the efficiency of protein translation from the target RNA.

In the protein synthesis method of the present invention, one example of the protein to be synthesized is an antibody, particularly synthesizing a light or a heavy chain or both of the antibody or single chain recombinant version of an antibody. The synthesis of the antibody is carried out preferably in an in vitro system or in an isolated cell system so that a functional nucleic acid molecule according to the present invention or aforementioned expression vector can be allowed to coexist, in the system, with a target RNA for which protein synthesis efficiency is to be increased.

### (Method for Treating Disease)

Whilst not part of the claimed invention, the protein synthesis efficiency-increasing method described herein can be used for treatment of a disease caused by a quantitative decrease in a predetermined normal protein, for example. In one aspect, there is provided the functional nucleic acid molecule of the claimed invention, the DNA molecule of the claimed invention, or the expression vector of the claimed invention, for use in medicine. In another aspect, there is provided the functional nucleic acid molecule of the claimed invention, the DNA molecule of the claimed invention, or the expression vector of the claimed invention, for use in the treatment of a haploinsufficiency or a disease that is caused by a quantitative decrease in a protein.

The disease is not especially limited, but may be, for example; myodegeneration such as muscular dystrophy; neurodegenerative disease such as Alzheimer's disease, Parkinson's disease, Amyotrophic Lateral Sclerosis, and triplet repeat diseases both at the protein encoding level (i.e. Huntington's disease) or at DNA/RNA level (i.e. Fragile X).

Furthermore, a tumor can be also applied to the present invention through the increase of expression of a pro-apoptotic protein or a tumor suppression protein for tumor treatment as for instance for p53 family members.

Such a tumor, including a cancer, may be any one of tumors including without limitation carcinoma, melanoma and sarcoma, bladder carcinoma, brain tumor, breast tumor, cervical tumor, colorectal tumor, esophageal tumor, endometrial tumor, hepatocellular carcinoma, gastrointestinal stromal tumor, laryngeal tumor, lung tumor, osteosarcoma, ovarian tumor, pancreatic tumor, prostate tumor, renal cell carcinoma, skin tumor, or thyroid tumor. The treatment method described herein but not claimed comprises the step of increasing the protein synthesis efficiency by the aforementioned protein synthesis efficiency-increasing method which comprises allowing a functional nucleic acid molecule according to the present invention or aforementioned expression vector to coexist, in a body of a subject, with a target RNA. In this step, the functional nucleic acid molecule itself or an aforementioned expression vector is transfected into a cell of the subject. The target RNA may be an endogenous RNA (mRNA or the like) in the cell of the subject or artificially synthesized RNA. Alternatively, the target RNA or a DNA molecule encoding the RNA may be transfected into the cell of the subject.

Further, the protein synthesis efficiency-increasing method according to the present disclosure is applicable to treatment of a disease by amplifying in a body of a subject a protein factor (e.g., interferon, an apoptosis-inducing factor, or the like) that ameliorates the disease. For example, an apoptosis-inducing factor amplified in accordance with the present disclosure can be effectively used for treatment of tumors or the like.

The treatment method described herein but not claimed may comprise the step of allowing a functional nucleic acid molecule according to the present invention or aforementioned expression vector to coexist with a target RNA encoding a protein that
ameliorates a disease in a body of a subject, wherein the target RNA hybridizes the antisense in the target determinant sequence of the functional nucleic acid molecule. In this step, the functional nucleic acid molecule itself or the aforementioned expression vector is transfected into a cell of the subject. The RNA encoding the protein that ameliorates the disease may be an endogenous RNA in the cell of the subject. Alternatively, the RNA encoding the protein that ameliorates the disease or a DNA molecule encoding the RNA may be transfected into the cell of the subject.

Any of the aforementioned treatment methods described herein but not claimed can be carried out as a pretreatment to an isolated cell to be transplanted into a body of the subject. The cell may be isolated from the body of the subject before treatment and transplantation. The aforementioned treatment methods may be a treatment method comprising the steps of (a) allowing a functional nucleic acid molecule according to the present invention or aforementioned expression vector to coexist in an isolated cell with a target RNA; and (b), after the step (a), transplanting the cell into a body of a subject. An isolated cell may be a cell which has the ability of differentiation, for example an Embryonic Stem cell (ES cell), an Embryonic Germ cell (EG cell), a somatic stem cell, especially a multi potent adult progenitor cell, stem cell, a hematopoietic stem cell, a vascular endothelia stem cell, a mesenchymal stem cell, a hepatic stem cell, a neural stem cell, an endothelial stem cell, a pancreatic stem cell, a primordial germ cell, or a multilineage-differentiating cell like a Muse cell (Kuroda et al., 2010, PNAS). Additionally, an isolated cell may be also an artificial undifferentiated cell, for example a nuclear transfer embryonic stem cell or a cell acquired the pluripotent ability by gene transfer and/or treatment of chemical compound, like an induced pluripotent stem cell (iPS cell (WO2007/069666, JP-A 2010-273680, JP-A 2010-284088, JP-A 2011-50379, JP-A 2011-4674, etc)). Additionally, an isolated cell may also be a fibroblast or an adult somatic cells that acquired the ability to become another somatic cells upon reprogramming. Additionally, after the step (a) but before the step (b), the isolated cell may be grown undifferentiated. Alternatively, after the step (a) but before the step (b), the isolated cell may be differentiated to obtain a differentiated cell or a group of differentiated cells (cell sheet or the like), and the differentiated cell or the group of differentiated cells may be transplanted into the body.

The subject indicates animals, wherein the animals include a human, preferably mammals including a human, more preferably a human. Further, the subject generally encompasses (a) one who has already shown the symptoms of a disease and (b) one who has a genetic predisposing cause but has not shown the symptoms of a disease yet. In view of this, the concept of "treatment" in the present invention includes therapeutic treatment and preventive treatment of a disease.

### [Example]

The present invention will be described below more specifically based on Examples, Comparative Examples, and the like. Note that the present invention is not limited to them.
Antisense transcription in synthenic PARK5/Uchl1 locus.

The FANTOM2 clone Rik6430596G22 was identified as a putative spliced AntiSense (AS) noncoding RNA (ncRNA9 of the Ubiquitin Carboxy Terminal Hydrolase 1 (UCHL1) gene 7 (Experiment 1). UchL1 is a neuron-restricted protein that acts as deubiquinating enzyme, ubiquitin ligase or monoubiquitin stabilizer (Reference 1; Liu, Y., Fallon, L., Lashuel, H.A., Liu, Z. & Lansbury, P.T., Jr. The UCH-L1 gene encodes two opposing enzymatic activities that affect alpha-synuclein degradation and Parkinson's disease susceptibility. Cell 111, 209-218 (2002)., Reference 2; Osaka, H., et al. Ubiquitin carboxy-terminal hydrolase L1 binds to and stabilizes monoubiquitin in neuron. Hum Mol Genet 12, 1945-1958 (2003).). It is mutated in an autosomal dominant form of PD (PARKS) (Reference 3; Leroy, E., et al. The ubiquitin pathway in Parkinson's disease. Nature 395, 451-452 (1998).). In Substantia Nigra (SN) of sporadic post-mortem brains UchL1 expression was found reduced and correlated to the formation of aSYN aggregates. Loss of Uchl1 activity has been also associated to Dementia with Lewy bodies (DLB) and Alzheimer's disease (AD). Increasing Uchl1 expression has been proposed as therapeutic strategy for AD since its ectopic expression rescued beta-amyloid-induced loss of synaptic function and contextual memory in a mouse model (Reference 1, Reference 4; Butterfield, D.A., et al. Redox proteomics identification of oxidatively modified hippocampal proteins in mild cognitive impairment: insights into the development of Alzheimer's disease. Neurobiol Dis 22, 223-232 (2006)., Reference 5; Castegna, A., et al. Proteomic identification of oxidatively modified proteins in Alzheimer's disease brain. Part II: dihydropyrimidinase-related protein 2, alpha-enolase and heat shock cognate 71. J Neurochem 82, 1524-1532 (2002)., Reference 6; Choi, J., et al. Oxidative modifications and down-regulation of ubiquitin carboxyl-terminal hydrolase L1 associated with idiopathic Parkinson's and Alzheimer's diseases. J Biol Chem 279, 13256-13264 (2004).).

Rik6430596G22 is a typical 5' head to head transcript that initiates within the second intron of Uchl1 and overlaps the first 72 nts of the Sense (S) mRNA including the AUG codon (Experiment 1). The non overlapping part of the transcript also contains two embedded repetitive sequences, SINEB2 and Alu, identified by Repeatmasker. The FANTOM2 cDNA clone spans a genomic region of 70kb and is a spliced transcript composed of four exons whose intron-junctions follow the traditional GT-AG rule (Experiment 2). In the remaining part of the disclosure Rik6430596G22 is referred as a natural AntiSense transcript to Uchl1 (AS Uchl1).

AS Uchl1 increases UchL1 protein levels with a mechanism that requires an embedded inverted SINEB2 repeat.

The interplay between S and AS transcripts was then examined. After cloning the full length cDNA for AS Uchl1 from MN9D cells with 5' rapid amplification of cDNA ends (RACE), a CMV-driven AS Uchl1 was transiently overexpressed in MN9D dopaminergic cells and endogenous Uchl1 mRNA and protein levels were monitored by qRT-PCR and western blotting. While no significant change in Uchl1 mRNA endogenous levels was observed, a strong and reproducible upregulation of UchL1 protein product was detected within 24 hours (Experiment 3). The inventors tested whether co-transfection of both cDNAs into HEK cells which do not express either transcript could recapitulate what was seen in MN9D cells. When increasing amounts of AS Uchl1 were co-transfected with murine Uchl1, a dose-dependent UchL1 protein upregulation was recorded in absence of any significant change in exogenous Uchl1 mRNA level (Experiment 4). This specific effect was not observed for unrelated controls such as GFP. To identify sequences and/or structural elements of AS Uchl1 mRNA that elicit its functional activity on UchL1 protein, deletion mutants were produced and tested in MN9D cells as well as in co-transfection in HEK cells. AS Uchl1 deletion constructs lacking the 5' first exon (AS Uchl1 Δ 5'), or the last three exons (AS Uchl1 Δ 3') failed to induce Uchl1 protein levels in both MN9D and HEK cell models, suggesting both 5' and 3' components were important to AS Uchl1 function (Experiment 5).

Additional deletion mutants were thus synthesized to assess the role of the embedded repetitive sequences, Alu and SINEB2, in UchL1 protein upregulation.

Targeted deletion of the region containing both the SINEB2 and Alu repeat elements (AS Uchl1 Δ SINEB2+ALU) prevented Uchl1 protein induction. Deletion of a single repetitive element, (Δ-SINE B2 (764-934) and Δ-ALU (1000-1045), revealed the SINEB2 was the functional region of the transcript required for UchL1 protein increase (Experiment 6). In all cases no change in Uchl1 mRNA level was detected by transfection of AS Uchl1 wild type and deletion constructs.

Additionally the Δ-SINEB2 mutant has a dominant negative activity on the full-length AS Uchl1.

Since the deletion mutant Δ-SINEB2 lacks 170 nucleotides potentially impairing AS Uchl1 RNA secondary structure, a mutant was produced with the SINEB2 sequence flipped in between nucleotide 764-934. Interestingly, SINEB2 flip was unable to increase UchL1 protein levels thus proving the orientation-dependent activity of the SINEB2 domain embedded within AS Uchl1 (Experiment 6).

### S/AS pairs with an embedded inverted SINEB2 repeat in the AS transcript identify a new functional class of ncRNA. :

The FANTOM3 collection of non-coding cDNAs was bioinformatically screened for other examples of natural AS transcripts that contain SINEB2 elements (B3 subclass) in the correct orientation and 5' head to head overlapping to a protein coding gene. This identified 31 S/AS pairs similar to the Uchl1/AS Uchl1 structure (Experiment 7).

To test whether the observation for Uchl1/AS Uchl1 generalizes to other examples, the AS overlapping transcript of Uxt (ubiquitously-expressed transcript), AS Uxt (Rik4833404H03) were cloned and over-expressed. Transfection of AS Uxt in MN9D dopaminergic cells showed up-regulation of Uxt protein product with no change in the total mRNA levels confirming a more general mechanism is at work (Experiment 8).

### AS Uchl1 is a nuclear-enriched transcript expressed in dopaminergic neurons. :

Multiplex RT-PCR on a panel of mouse adult tissues, macroscopically dissected brain regions and neuronal cell lines found that AS Uchl1 expression was restricted to ventral midbrain, cortex and MN9D dopaminergic cells but absent in non-neuronal tissues, and cell lines. Double in situ hybridization with riboprobes targeting the non-overlapping region of Uchl1 showed that the mRNA was prevalent in the cytoplasm of cells of the hippocampus, cortex and subcortical regions as well as of the dorsal and ventral midbrain. AS Uchl1 riboprobe decorated similar structures. A combination of double in situ hybridization with anti-tyrosine hydroxylase immunohystofluorescence showed that mRNAs for Uchl1 and AS were expressed in the same DA neurons of the SN. Intriguingly, transcripts for the S/AS pair were prevalently localized in two different subcellular compartments: mature Uchl1 mRNA was mainly observed in the cytoplasm, while the AS RNA was nuclear, accumulating in specific subnuclear regions (Experiment 9). 50% of cellular transcripts have been recently found enriched in the nucleus representing mainly ncRNAs with unknown function. Nuclear-retained RNAs tend to accumulate in areas, called paraspeckles, that strongly resemble sites of AS Uchl1 localization and which association is regulated by embedded SINEs (Reference 7; Chen, L.L., DeCerbo, J.N. & Carmichael, G.G. Alu element-mediated gene silencing. Embo J 27, 1694-1705 (2008).).

By taking advantage of RACE, the precise transcriptional start site (TSS) of the AS Uchl1 gene was mapped in MN9D cells. As shown in Experiment 2, the TSS lies 250 bps upstream the previously annotated sequence and is localized in the second intron of Uchl1.

### AS Uchl1 is down-regulated in PD neurochemical models and human post-mortem brains. :

A 70-kb region of the mouse genome encompassing the AS UchL1 locus was then compared to the corresponding human genomic sequence using Genome Vista alignment (http://genome.lbl.gov/cgi-bin/GenomeVista). By the use of primers designed on the human sequence in correspondence to CST peaks, a 1.6 kb non-coding transcript, 5' head to head AS to human UCHL1 gene, was cloned from human brain RNA. The anatomical organization of hAS UCHL1 gene was very similar to its mouse counterpart including the extension of the S/AS pair overlapping region as well as the presence of embedded repetitive elements. hAS UCHL1 expression was highly restricted to neuronal tissues as found for mouse.

UCH-L1 protein synthesis is increased upon rapamycin treatment through nucleus-cytoplasmic shuttling of AS Uchl1 RNA and AS-dependent recruitment of Uchl1 mRNA to active polysomes.

So far, AS ncRNA is able to increase S protein levels with no change in the quantity of S mRNA. Since in physiological conditions S mRNA and AS ncRNA seem to be localized in different subcellular compartments, several stressors that have been implicated in PD pathogenesis for their ability to redistribute the nuclear AS ncRNA into the cytoplasm, where translation takes place, were assayed. MN9D cells were exposed to hydrogen peroxide 1mM, serum starvation, rapamycin 1 ug/ml, tunycamycin 20nM and TNFalpha 20 nM for 45 minutes and AS Uchl1 mRNA content was independently measured in the cytoplasm and nucleus by qRT-PCR. The majority of treatments had no effect, however rapamycin strongly up-regulated the amount of AS Uchl1 cytoplasmic mRNA (Experiment10).

Rapamycin is a well known inhibitor of CAP-dependent translation through its effect on mTORC1 and subsequent repression of S6K and 4E-BP1 activities. It is currently tested as anti-cancer drug and proposed for clinical trials for neurodegenerative diseases. Block of translation initiation mediated by rapamycin is able to rescue DA cell loss observed in knock-out flies for parkin and pink1 as well as in those over-expressing the dominant PD-associated mutation of LRRK2 (Reference 8; Tain, L.S., et al. Rapamycin activation of 4E-BP prevents parkinsonian dopaminergic neuron loss. Nat Neurosci 12, 1129-1135 (2009).). Furthermore, it protects mammalian DA cells from neurochemical intoxication in vitro and in mice (Reference 9; Malagelada, C., Jin, Z.H., Jackson-Lewis, V., Przedborski, S. & Greene, L.A. Rapamycin protects against neuron death in in vitro and in vivo models of Parkinson's disease. J Neurosci 30, 1166-1175 (2010)., Reference 10; Malagelada, C., Ryu, E.J., Biswas, S.C., Jackson-Lewis, V. & Greene, L.A. RTP801 is elevated in Parkinson brain substantia nigral neurons and mediates death in cellular models of Parkinson's disease by a mechanism involving mammalian target of rapamycin inactivation. J Neurosci 26, 9996-10005 (2006).). Recently, rapamycin was shown to prevent L-DOPA-induced dyskinesia, a common severe motor side effect of the symptomatic treatment for PD (reference 11; Santini, E., Heiman, M., Greengard, P., Valjent, E. & Fisone, G. Inhibition of mTOR signaling in Parkinson's disease prevents L-DOPA-induced dyskinesia. Sci Signal 2, ra36 (2009).).

The effects of rapamycin on the cytoplasmic content of AS Uchl1 were confirmed by the presence of a concomitant decrease in its nuclear steady state levels, and by the absence of any de-novo transcription of AS Uchll (Experiment 10). Total cellular content of these transcripts remained constant. Uchl1 mRNA showed no changes in subcellular distribution, de novo transcription or total cellular content.

Despite the block in CAP-dependent translation, upon rapamycin treatment UchL1 protein level increased several fold (Experiment 11).

The inventors assessed whether AS Uchl1 was required for UchL1 induction. Stable MN9D cell lines were then established expressing constitutively shRNA for AS Uchl1, targeting the AS Uchl1 promoter region from -4 to + 15 nt around the RACE-validated TSS (Reference 12; Hawkins, P.G., Santoso, S., Adams, C., Anest, V. & Morris, K.V. Promoter targeted small RNAs induce long-term transcriptional gene silencing in human cells. Nucleic Acids Res 37, 2984-2995 (2009)). As expected, scrambled cells showed UchL1 protein up-regulation as in MN9D parental line while cells expressing shRNA for AS Uchl1 lacked any changes in UchL1 protein levels proving a causal link between rapamycin induction of Uchl1 protein and AS Uchl1 ncRNA expression (Experiment 12). As independent model, stable cell lines with expression of a dominant negative mutant of AS Uchl1(Δ-SINEB2) were established. When control MN9D cells stable for empty vector were treated with rapamycin, UchL1 protein was found increased. In presence of the dominant negative form of AS Uchl1 this upregulation was no longer visible (Experiment 13).

### A model for AS-dependent increase in S-encoded protein levels upon rapamycin treatment. :

In growing cells mTORC1 signaling is required for proliferation and controls CAP-dependent translation machinery through the phosphorylation of its downstream substrates 4E-BPs and S6K. The cytostatic drug rapamycin inhibits mTORC1 activity leading to block of CAP-dependent translation. Here, the inventors show that in these conditions AS transcription is required for protein synthesis of selected mRNAs. Upon rapamycin addition, the nuclear-enriched ncRNA AS Uchl1 is transported into the cytoplasm where it recruits mRNAs of its S protein-encoding partner to polysomes for translation.

AS Uchl1 is thus the representative member of a new functional class of ncRNAs that are associated to S/AS pairs in the mammalian genome and appears to be composed by two domains. The overlapping region at the 5' provides specificity to a protein-encoding mRNA partner transcribed from the complementary strand. An inverted SINEB2 element at 3' is required for translational activation, representing a new function for embedded SINEB2 in the cytoplasm.

The manipulation of Uchl1 expression in vivo has been proposed for therapeutic intervention in neurodegenerative diseases, including PD and AD. Natural AS transcripts with embedded repetitive elements may thus represent endogenous molecular tools to increase protein synthesis of selected mRNAs defining a potential new class of RNA therapeutics.

Rapamycin is currently under intense scrutiny in biomedical research both as neuroprotective agent for neurodegenerative diseases and as anti-cancer drug. In mice rapamycin prevents L-DOPA-induced dyskinesia, a common severe motor side effect of the symptomatic treatment for PD.

Furthermore, it protects neurons from apoptosis both in Drosophila genetic models as well as upon neurochemical intoxication in mammals making it an attractive molecule for anti-parkinsonian therapies (Reference 11; Santini, E et al., (2009)). It is thus important to better understand its modes of action in vivo and its interplay with pathways involved in familiar cases of PD, as shown here for Uchl1. The role of AS transcription in rapamycin-induced protein synthesis adds an unexpected switch to its activities.

### Artificially synthesized AS for increasing EGFP expression or antibody expression up-regulates the expression of each target in the cell. :

The inventors tested whether or not increased protein synthesis can be achieved on a synthetic RNA by inserting a 72 nt long target determinant sequence antisense to the enhanced green fluorescent protein (EGFP) into the appropriate sequence in AS Uchl1. As in Experiment 14, the expression of the artificial AS for up-regulating EGFP expression was designed. The fragments encoding the artificial AS was cloned into pcDNA3.1- vector (Invitrogen). This proves that a functional nucleic acid molecule with a target determinant sequence and a regulatory sequence can increase protein synthesis efficiency of any gene of interest.

In the same manner, the antibody can be applied as shown in Experiment 15. An artificial AS for up-regulating a recombinant antibody was designed to target the leader sequence of the recombinant antibody (overlap is 72bp around the ATG) and embedded into the vector (AS Fc). An artificial AS comprising a scramble sequence as the target determinant sequence (Scrambled) was also produced as control (Fig. 15). The target sequence of the antibody was included in pHYGRO vectors (pHYGRO). HEK cell line was co-transfected with pHYGRO and AS Fc or control. Transient transfection in HEK cells produced an AS Fc specific upregulation of the recombinant antibody in cell lysates. AS Fc increases the encoded protein levels in transfected HEK cells while a scramble overlapping sequence or an empty plasmid does not, detected through the SV5 tag.

For further examination (Experiment 16), HEK cell line was transfected with EGFP expression vector pEGFP-C2 (Clontech) with Lipofectamine 2000 (Invitorgen) in accordance with manufacture's instructions. Then, the transfectants which stably express low EGFP expression were selected. AS GFP comprises target determinant sequence against EGFP and it was artificially designed to increase the efficiency of EGFP protein synthesis (Fig. 16). The stable transfectants were further transfected with the vector encoding AS GFP or control vector. After 24 hours or 48 hours from the transfection of AS GFP construct or control vector, the cells were collected and lysed. The GFP level in the cell lysate was monitored by western blotting. The low expression of EGFP in the transfectant was increased by transfection of the AS GFP construct (Fig. 16).

In an additional experiment, a construct with a target determinant sequence that overlaps for 44nt to the plasmid backbone till the ATG codon for EGFP has been synthesized. This contruct (SINEup005) increases protein synthesis of the target gene very efficiently (more than 10 fold).

Therefore, this is one of evidences that the new type of AS which the inventors found can be artificially designed as long as the functional nucleic acid molecule includes a target determinant sequence comprising an antisense sequence to a target sequence in the protein-encoding RNA for which protein synthesis efficiency is to be increased and a regulatory sequence having an activity of increasing of the protein synthesis efficiency.

### Methods

### Plasmids

RACE fragment : The 5' UTR of AS Uchl1 was amplified by RACE PCR (GeneRacer, Invitrogen) by MN9D total RNA and cloned into pGEM^{®}-T Easy vector (Promega).

Full length AS Uchll : Full length DNA sequence of AS was amplified via fusion PCR starting from RACE fragment and FANTOM clone Rik 6430596G22 with following primers For mAS Uchl1 fl 5'-ACAAAGCTCAGCCCACACGT- 3' (SEQ ID No:13) and Rev mAS Uchl1fl 5'-CATAGGGTTCATT -3'(SEQ ID No: 14).

Uchll : Mouse Uchl1 mRNA was cloned from FANTOM 2900059022 with following primers: For mUchl1 5'-ATGCAGCTGAAGCCGATG-3'(SEQ ID No:15) and Rev mUchl1 5'-TTAAGCTGCTTTGCAGAGAGC-3' (SEQ ID No:16) AS Uchl1 shRNA : Oligo containing the sequence -14/+4 around the TSS of AS Uchl1 CGCGCAGTGACACAGCACAAA (SEQ ID No:17) are cloned into pSUPER.retro.puro vector (OligoEngine, Seattle, WA), scrambled sequence was used as control.

### Deletional mutants :

Δ5': For mAS Uchl1 fl and Rev Δ 5'AS Uchl15' TACCATTCTGTGCGGTGCA-3' (SEQ ID No:18).

A3': For mAS Uchl1 GACCTCCTCTAGCACTGCACA-3' (SEQ ID No:19) and Rev mAS Uchll fl.

For fine deletional mutants, PCR fragment I is cloned NheI-EcoRI site in PcDNA3.1- and PCR fragment II into following EcoRI-HindII site.

### AS Uchl1 Δ ( Alu+SINEB2) :

PCR fragment I: For mAS Uchl1 fl and Rev pre-SINE B2 5'-CAATGGATTCCATGT-3' (SEQ ID No:20). PCR fragment II: For post-ALU 5'-GATATAAGGAGAATCTG-3' (SEQ ID No:21) and Rev mAS fl.

### AS Uchl1 Δ(Alu) :

PCR fragment I: mAS Uchl1 fl and Rev pre-Alu 5'- TTATAG TATGTGTTGTC-3' (SEQ ID No:22). PCR fragment II: For post-ALU 5'-GATATAAGGAGAATCTG-3' (SEQ ID No:23) and Rev mAS fl cloned into EcoRI-HindII site.

### AS Uchl1 Δ (SINEB2) :

PCR fragment I: For mAS Uchl1 fl and and Rev pre-SINE B2 5'-CAATGGATTCCATGT-3' (SEQ ID No:24). PCR fragment II: For post-SINE B2 5'- GAATTCCTCCAGTCTCTTA -3' (SEQ ID No:25) and Rev mAS fl.

AS uchl1 (Alu+SINEB2) flip : PCR fragment I: obtained with For SINE B2 inside 5'-TGCTAGAGGAGG-3' (SEQ ID No:26) and Rev Alu flip 5'- GTCAGGCAATCC -3' (SEQ ID No:27) are cloned in the unique EcoRI site of AS Uchl1 Δ ( Alu+SINEB2). AS uchl1 SINEB2 flip : PCR fragment obtained with For SINE B2 inside 5'-TGCTAGAGGAGG-3' (SEQ ID No:28) and Rev SINE flip 5'-AAAGAGATGGC-3' (SEQ ID No:29) are cloned in the unique EcoRI site of AS Uchl1 Δ (SINEB2).

### Cells

MN9D cells were seeded in 10 mm petri-dishes in Dulbecco's modified Eagle's medium containing 10% of fetal bovine serum and, penicillin (50 units/ml), streptomycin (50 units/ml). Treatments were done by adding Rapamycin (R0395, Sigma) at final concentration of 1ug/ml in fresh medium for 45 minutes.

For the establishment of MN9D stable cells (siRNA -15/+4, siRNA scrambled, pcDNA 3.1- and Δ SINE B2) MN9D cells were seeded in 100mm petri-dishes and transfected with Lipofectamine 2000 (Invitrogen) according to manufacturer's instruction, the day after cells seeded for selection with 500uM of Neomycin (#N1142, Sigma). HEK-293T cells were grown in DMEM (GIBCO) supplemented with 10% fetal bovine serum (Sigma-Aldrich), 100 units/mL penicillin, and 100 µm/mL streptomycin (Sigma) at 37 °C in a humidified CO₂ incubator.

### PCR

PCR analysis: Total RNA was extracted using Trizol reagent (Invitrogen) according to manufacturers instruction. It was subjected to DNAse I treatment (Ambion) and 1 ug was retrotranscribed using iScript cDNA Synthesis Kit (BioRad). Real Time qRT-PCR was carried out using Sybr green fluorescence dye (2X iQ5 SYBR Green supermix, BioRad). Actin and GAPDH were used as internal standard. Relative quantification was performed with the comparative Ct method.

Actin: sense 5'- CACACCCGCCACCAGTTC-3' (SEQ ID No:30), antisense 5'-CCCATTCCCACCATCACACC-3' (SEQ ID No:31). Gapdh: sense 5'-GCAGTGGCAAAGTGGAGATT-3' (SEQ ID No:32), antisense 5'-GCAGAAGGGGCGGAGATGAT-3' (SEQ ID No:33).

AS Uchl1 overlap: sense 5'-GCACCTGCAGACACAAACC-3' (SEQ ID No:34), antisense 5'-TCTCTCAGCTGCTGGAATCA-3' (SEQ ID No:35).

AS Uchll : S'CTGGTGTGTATCTCTTATGC (SEQ ID No:36) antisense S'CTCCCGAGTCTCTGTAGC (SEQ ID No:37). Uchll : sense 5'- CCCGCCGATAGAGCCAAG (SEQ ID No:38), antisense 5'-ATGGTTCACTGGAAAGGG-3' (SEQ ID No:39). ASUchl1 pre RNA : 5'-CCATGCACCGCACAGAATG-3' (SEQ ID No:40), antisense 5'-GAAAGCTCCCTCAAATAGGC-3' (SEQ ID No:41).

Pre_A0ribosomal RNA : sense 5'-TGTGGTGTCCAAGTGTTCATGC-3' (SEQ ID No:42), antisense5'-CGGAGCACCACATCGATCTAAG-3 (SEQ ID No:43).

AS_Uxt: sense5'-CAACGTTGGGGATGACTTCT (SEQ ID No:44), antisense5'-TCGATTCCCATTACCCACAT (SEQ ID No:45);

Uxt: sense5'-TTGAGCGACTCCAGGAAACT-3' (SEQ ID No:46), antisense 5'-GAGTCCTGGTGAGGCTGTC-3' (SEQ ID No:47).

Multiplex RT PCR was performed with SuperScript^{®} III One-Step RT-PCR System with Platinum^{®} Taq DNA Polymerase (Invitrogen). 500 mg of total DNAse traeated RNA was incubated with reverse primers for Gapdh, Uchl1, AS Uchl1. The reaction was performed for 60 minutes at 60 degrees.

Each volume was then splitted in three and forward primers were added at final concentration of 200nM to the reaction. The PCR reaction comprised 40 cycles of 95 degrees for 15 seconds, followed by 60 degrees for 45 seconds, and final 68 degrees for 30 seconds.

### Western blot

Cells were lysed in SDS sample buffer 2X. Proteins were separated in 15% SDS-polyacrilamide gel and transferred to nitrocellulose membrane. Immunoblotting was performed with the primary antbodies: anti-Uchl1 (#3524 Cell Signalling) 1:300 and anti-b actin (A5441, Sigma) 1:5000. Signals were revealed after incubation with recommended secondary antibodies conjugated with horseradish peroxidase by using enhanced chemioluminescence for Uchl1 (#WBKLS0500 Immobilion Western Chemioluminescent HRP substrate) and ECL detection reagent (RPN2105, GE Healthcare).

### Cellular fractionation

Nucleo cytoplasmic fractionation was performed using Nucleo Cytoplasmic separation kit (Norgen) according to manufacturer's instruction. RNA was eluted and DNAseI treated. The purity of the cytoplasmic fraction was confirmed by Real Time qRT-PCR on Pre-ribosomal RNA.

### Two colour In situ hybridization (ISH)

Reagents: anti DIG antibody D8156 (Sigma); streptavidin HRP RPN1231-100UL (Amersham Bioscience); DIG labeling mix #11 277 073 910 (Roche) BIO-labelling Mix #11 685 597 910 (Roche), Ribonucleic acid, transfer from baker's yeast R8759 (Sigma), Deoxyribonucleic acid, single stranded from salmon testes D7656 (Sigma), Blocking reagent #11 096 176 001 (Roche), *TSA Cy3 system* (Perkin Elmer, Heidelberg, Germany).

After perfusion with 4% formaldehyde, the mouse brain was cryoprotected overnight in 30% sucrose. *In situ* hybridization was performed on cryostat slices (16 um). Sense and antisense probes were generated by *in vitro* transcription from the cDNA encoding the distal 600 bps of mouse Uchl1 cDNA and the last 1000 bps of mouse AS Uchl1. The probes for Uchl1 were labeled with digoxigenin, probes for AS Uchl1 were labeled with biotin. Incorporation of both bioting and digoxigenin was checked via Northern Blot. Slices were pretreated with hydrogenum peroxide 3% for 30 minutes. Hybridization was performed with probes at a concentration of 1 mg/ml (Uchl1) and 3 mg/ml for AS Uchl1 at 60°C for 16 h. For biotinilated RNA detection, streptavidin-HRP was used 1:250 for 2 hours in TNB buffer (Tris HCl PH 7,5 100mM, NaCl 150 mM, 0,5% Blocking Reagent), and signals are visualized using the *TSA Cy3 system* after washing in TNT buffer (Tris HCl PH 7,5 100mM, NaCl 150 mM, 0,05% tween).

ISH on DIG-labeled probe was performed by incubating slices with monoclonal anti-DIG antibody after TSA reaction. To combine RNA ISH with immunofluorescence, slice were incubated with the antibody anti TH (#AB152, Chemicon) 1:1000. Signals are then detected with fluorescent dye-conjugated secondary antibody goat anti-rabbit 405 and goat anti-mouse 488. Sections were then washed, mounted with Vectashield (Vector lab) mounting medium and observed at confocal microscope (Leica).

### Post-mortem human brain samples

Brain samples were obtained from the brain bank at the Institute of Neuropathology, Bellvitge Hospital (University of Barcelona, Spain). Samples were dissected at autopsy with the informed consent of patients or their relatives and the institutional approval of the Ethics Committee of the University of Barcelona. Brains were obtained from Caucasian, pathologically confirmed PD cases and age-matched controls (Navarro et al., 2009). Briefly, all cases of PD had suffered from classical PD, none of them had cognitive impairment and their neuropathological characterization was made according to established criteria. Control healthy subjects showed absence of neurological symptoms and of metabolic and vascular diseases, and the neuropathological study disclosed no abnormalities, including lack of Alzheimer disease and related pathology. The time between death and tissue preparation was in the range of 3 to 5 hours.

### Bioinformatic analysis

For the identification of a cadidate human hortologue of AS Uchl1, conservation between human and mouse in the orthologus region of AS uchl1 was performed using VISTA genome browser. The inventors selected parameters for conserved sequence tags (CTS) that have a minimum of 75% identity between the mouse and human genome. For each conserved element a primer on the homologus human region was designed.

For the identification of additional translational activator candidates, the inventors searched for FANTOM3 full-length cDNAs that were non-coding RNAs and overlap the 5' end of coding transcripts in a head to head configuration [PMID: 16141072]. The filtered set of 8535 FANTOM3 ncRNA transcripts described in the Nordstrom et al 2009 (Nordstrom, K.J., et al. Critical evaluation of the FANTOM3 non-coding RNA transcripts. Genomics 94, 169-176 (2009).) was used as our starting point. Genomic locations of these ncRNA transcripts and REFSEQ (Maglott, D.R., Katz, K.S., Sicotte, H. & Pruitt, K.D. NCBI's LocusLink and RefSeq. Nucleic Acids Res 28, 126-128 (2000)) coding transcripts were extracted from the alignments in the UCSC Genome browser (Kent, W.J., et al. The human genome browser at UCSC. Genome Res 12, 996-1006 (2002)) to identify a set of 788 coding-sense: non-coding-antisense pairs. The ncRNAs were then checked by repeat masker to identify SINEB2 related sequences (Smit, AFA, Hubley, R & Green, P. RepeatMasker Open-3.0.1996-2010 <http://www.repeatmasker.org>). This reduced the number of pairs to 127 protein coding transcripts with overlap at the 5' end (60 with a sense strand version of the repeat, 53 with an antisense version and 14 with both sense and antisense versions).

Alignment of the SINEB2 related elements was then carried out using Clustalw (http://www.ebi.ac.uk/Tools/clustalw2/index.html.

From this analysis the antisense overlapping transcripts with a repeat most like that of Uchl1-as and in the same orientation were chosen for experimental testing (Uxt1-AS).

### Sequences

As example, the following is a list of sequences that are complementary to protein coding mRNAs. They contain a fraction that provides examples of a target determinant sequence [now in light blue highlight] and a regulatory sequence [how highlighted in red]. The regulatory sequence in this list of natural antisense is as short at 89 nucleotides in this example. The length of the adaptor sequences in this partial list of antisense RNAs is as short as 44 nt.

Below each sequence there is a summary of the alignments with the retrotransposon elements as determined by Repeatmask program.
AK078321 (AS Uchll)
AK078321.1 (SEQ ID No:7) 
aaacgatgctcttggaggatagggacagagactgcgcgccgcgccactcactttgttcagcatctgaaagccaaa
agcaaagaggaaaatgataataaaactaaatgattcagctaccgagctgtagctaagggtcagccttatttctcc
cgaagcgacccagcagctatgcttacctcggggttaatctccatcggcttcagctgcatcttcgcggatggcacc
tgcagacacaaacccgaggagccgaaaaacagccggtggagccgcccaggctgctgttataaagcgccggcctc
gctcactgggaaagcctgagcaggggagacgggagcagaaacaagcagagcaggaaggccaagagggctcgaact
cccccatgcaccgcacagaatggtacaagccaagcccccaaaccttgcagtctcactcgccgaagtgctcccccg
actgggcatggtagcacgcacctgtgattccagcagctgagagagaggccgagcccacatggaatccattgtgca
gtgctagaggaggttagaagagggcattggatcccttagaatggagttatacggtaatttgtggtggttgtga
accaccatgtggatggatattgagttccaaacactggtttgtgtaagagcatccagtgctttaagtgctgagc
catctctttagtttagtctcttaaaaaacaaacaaacgaacgaacagcaagggagctgggtatgataacacata
ctataattctagtattcaggatgctgaaacaggaggattgcctgactgggagatataaggagaatctgttgtcac
ccccacccctccccataaaggcagaataaaagaacgtcctataaacaaataaacaaacaacccaataaaacaaaa
ccaagatctctccacctttcttgcttttcagactttgtaataaggccctttggagtgcaggatattcggcag
gacaagcagagagggagaccatcagttctttctttgatcaagaagactatgttccttagcaaactggtgtgtatt
atctcttatgcaatgagcctggaaagagggcacagccaccgaggatggtacagcatggatggatggtacgctaca
gagactcgggagcccaactgtgagtggctgactggcatggtaggttcagggaagaattggcctgtgaagaaaatg
ttcttgaaaagtgaataaggtgcaggaggtaggagtgggtcctgggcaaagcaggggggtgcatcccagcctcagg
gaatagcacagcagaggtctgttgatgcatgcgagtgcatgacctgcttgccaatagacgatcaagaatggcaa
agcatcatgggtgatgagtgggagaggggatgagacattcctttctcccctgctgagacttccattgaaccgatga
gttctgaatagaagatgcccccccacccccccaccagtgtagaatctgaaggaggcatatattaccctatatta
ctctgtgttggcggcgagctatctgacagccaaccttcccatacatttcattgggcatacactaatgacaggaag
ttcctttttgcttgtatgcaagagatggctcacacgatggagaatttaatcttgttagtttgttatttatgtgtcc
taaattttgttcaataaaaatgaaacactcctatg
**In Bold overlap region (UchL1) *Italic is 72 b*p**
**The boxed nucleotide CAT is complementary to the CDS starting ATG** 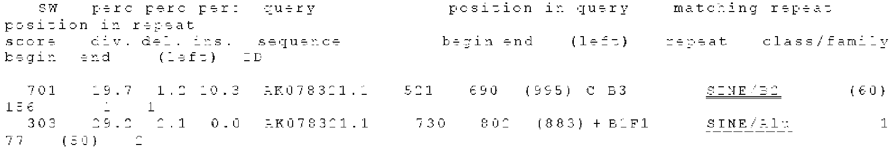
**ANNOTATION EVIDENCE:** 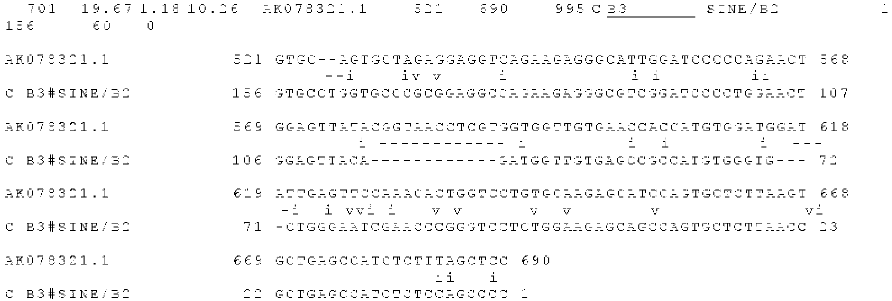
Matrix = 25p43g.matrix
Transitions / transversions = 2.00 (20 / 10)
Gap_init rate = 0.02 (3 / 169), avg. gap size = 6.00 (19 / 3)
AK029359.1 (AS Uxt) (SEQ ID No:8) 
tagtctcgctgcagtacgtcactgataaagccactcaacgttggggatgacttctttggctcgagctacggagt
tgcttctgcccaacccgatttggaactcgttcactcaccggtacaggagtacggcgtttaccgtagggccaccg
gcaataactgccagaagaagttgtgggattccctggaactggagcaaccaacagtttgtgtgcaccatgtgggta
atggcaatcgaacttggtccctataagactttctagtgttttaactactgagttgtatttttttcggaattg
attcacataactattatgctatactgtttgaagtatttattagaaaaacatcagaaagagatttggaccactttc
atttacatgaagaaatatcttagggttctttttcaggtatctttgagtatcttctgacac[?]agaagatcctgtaa
actctacagacttcaacagaattgaagaacctggacagagcagagttaccaacaagagagcccagggatagcatt
aacatggtggttactcaagacctaactcagccagggagacataccaaggcctatgaggtgaagggaaaagaaggg
tgacccaaagggcaggaatttttatcctgaacttttgagccttatagaaaaacataatttgttgggcatgtttt
ctgaccctcatacagtttttacaaccatttgaagatatagttctagctctagtaggctctacaggaaggtatatc
aaactttttaacagaacactttatttatttaaatatatgagtatttcacctgcataggtgcatagtacccacaga
gactagaagagggtggcagatctcctgagactggagttaatgtttgtgagctgccatgtggatgttgaaatcaa
acccaggtcctttggaaggcaggcaggtgctcttaattatggaagcatctcttcagcttttaccctagttcttta
atttgttttaaatttttgaggtagggtttgctaagttacttgggctggccttggactagtaaccattctgtgt
cagccttctgggattagaggtatgtgctagcatgtttagcatctttctatttctttggtttccttctaattaat
taaaaaatacattatattct
**In Bold overlap region (UchL1)** 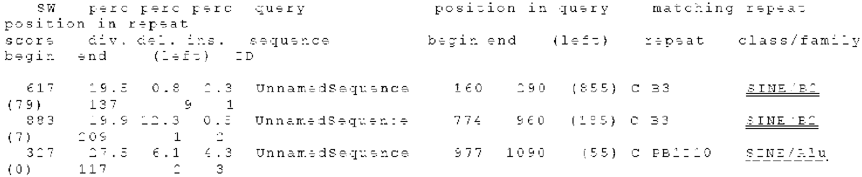
**ANNOTATION EVIDENCE:** 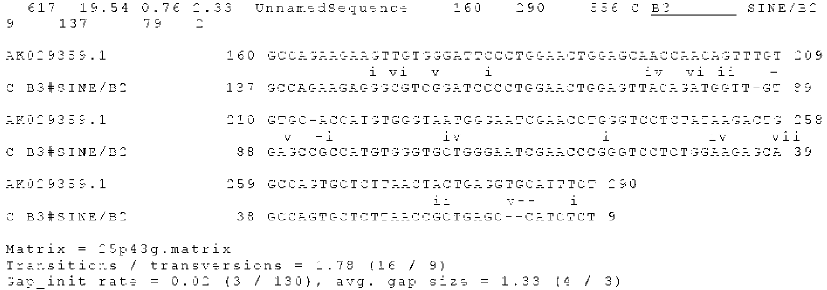
**ANNOTATION EVIDENCE**:

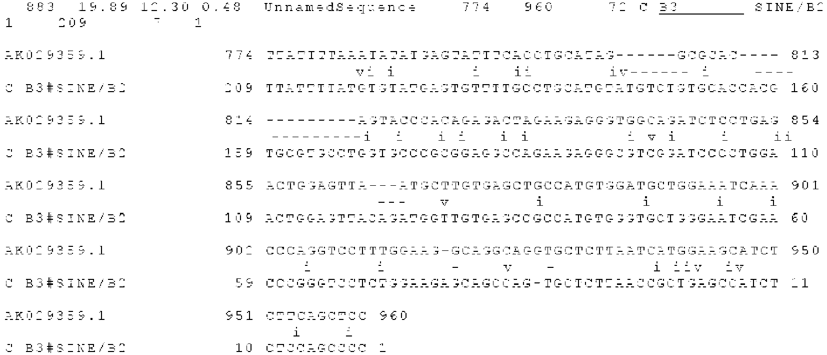

### Other sequences in the study:

B2#SINE/B2 (SEQ ID No:9) 
gggctggagagatggctcagtggttaagagcacctgactgctcttccagcggtcctgagttcaattcccagcaaccacat
ggtggctcacaaccatctgtaatgagatctgatgccctcttctggtgtgtctgaagacagctacagtgtacttacatata
ataaataaataaataaataaatcttaaaaaaaaaaaaaagaaagaaaaa
 >B3#SINE/B2 216 bp (SLO ID No: 10) 
GGGGCTGGAGAGATAGCTCAGCGGTTAAGAGCACTGGCTGCTCTTCCAGAGGACCCGGGTTCGGTTCCCAGCACCCACAT
GGCGGCTCACAACCGTCTGTAACTCTAGTTCCAGGGGATCTRACNCCCTCTTCTGACCTCCACGGGCACCAGGCACGCAC
GTGGTACACAGACGTACATGCARGCAAAACACTCATACACATAAAATAAAAATAAATMTTTWAAAAAA
**Uchl1** cloned ito Pme-blunt site of pcDNA 3. 1-(SEQ ID No: I1) 
CGGCTCCTCGGGTTTGTGTCTGCAGGTGCCATCCGCGAAGATGCAGCTGAAGCCGATGGAGATTAACCCCGAGATGCTGA
ACAAAGTGTTGGCCAAGCTGGGGGTCGCCGGCCAGTGGCGCTTCGCCGACGTGCTAGGGCTGGAGGAGGAGACTCTGGGC
TCAGTGCCATCCCCTGCCTGCGCCCTGCTGCTCCTGTTTCCCCTCACGGCCCAGCATGAAAACTTCAGGAAAAAGCAAAT
TGAGGAACTGAAGGGACAGGAAGTTAGCCCTAAAGTTTACTTCATGAAGCAGACCATCGGAAACTCCTGTGGTACCATCG
GGTTGATCCACGCAGTGGCCAACAACCAAGACAAGCTGGAATTTGAGGATGGATCCGTCCTGAAACAGTTTCTGTCTGAA
ACGGAGAAGCTGTCCCCCGAAGATAGAGCCAAGTGTTTCGAGAAGAACGAGGCCATCCAGGCGGCCCATGACTCCGTGGC
CCAGGAGGGCCAGTGTCGGGTAGATGACAAAGTGAATTTCCATTTTATTCTGTTCAACAACGTGGACGGCCATCTGTACG
AGCTCGATGGGCGAATGCCCTTTCCAGTGAACCATGGCGCCAGCTCAGAGGACTCTCTGCTGCAGGATGCTGCCAAGGTC
TGCAGAGAATTCACTGAGCGCGAGCAGGGGGAGGTCCGCTTCTCTGCCGTGGCTCTCTGCAAAGCAGCTTAAGTCTGGGG
AGAGAGAACCAGCCGATCCCCCCTTCCCTGGGCAGGTGCGCGCGGCCCGCCCTTGGTTTGCAGCTTTAGCACTTAGAACC
ACAGCTGTCTTCTTGCGTTCTACAGCCCCATCCCCTCCACCCCACCCAGGCCACCAGGGGGCTCTGTCACAGCCACACCA
GGCTGAGCACTTTCCCTCCTGTGTGTCTCGTACCTTGCTCTCTACGGTCTCTTGGTTTCTGTCTGTAAGTTACGGCCCT
GGATGTGGTTTGTCTAGTCCTTAAGAGGAAGAATAAAACTTTGCTGGTGAGAG
AS Uchl1 full length cloned into Xba-HindlI site in pcDNA3. 1-(SCO ID No: 12)

ACAAAGCTCAGCCCACACGTGGCTCGCGCGAAGCCCTCGGACTAGAGTCCGCGGCCGTCGCCACGCCCTCGAGAGCTGC
TCCCCGCGCTCGTTGCTGTCCGGGTCCATCCTCCGCCACCTCCCCGTGATCGATCTCTCAGTCACTCCCAAACCCCTAGA
TAACTCAGGGCAGAGACGACACCCAGCTGGGCGcCCCAGCCCAGCCACCTCCATTGCACAGGGCGCGGCCGGCTGGGCGT
CTCCAAACGATGCTCTTGGAGGATAGGGACAGAGACTGCGCGCCGCGCCACTCACTTTGTTCAGCATCTGAAAGCCAAAA
GCAAAGAGGAAAATGATAATAAAACTAAATGATTCAGCTACCGAGCTGTAGCTAAGGGTCAGCCTTATTTCTCCCGAAGC
GACCCAGCAGCTATGCTTACCTCGGGGTTAATCTCCATCGGCTTCAGCTGCATCTTCGCGGATGGCACCTGCAGACACAA
ACCCGAGGAGCCGAAAAACAGCCGGTGGAGCCGCCCAGGCTGCTGTTATAAAGCGCCGGCCTCGCTCACTGGGAAAGCC
TGAGCAGGGGAGACGGGAGCAGAAACAAGCAGAGGAGGAAGGCCAAGAGGGCTCGAACTCCCCCATGCACCGCACAGAAT
GGTACAAGCCAAGCCCCCAAACCTTGCAGTCTCACTCGCCGAAGTGCTCCCCGGACTGGGCATGGTAGCACGCACCTGTG
ATTCCAGCAGCTGAGAGAGAGGCCGAGCCCACATGGAATCCATTGTGCAGTGCTAGAGGAGGTCAGAAGAGGGCATTGGA
TCCCCCAGAACTGGAGTTATACGGTAACCTCGTGGTGGTTGTGAACCACCATGTGGATGGATATTGAGTTCCAAACACTG
GTCCTGTGCAAGAGCATCCAGTGCTCTTAAGTGCTGAGCCATCTCTTTAGCTCCAGTCTCTTAAAAAACAAACAAACGAA
CGAACAGCAAGGGAGCTGGGTATGACAACACATACTATAATTCTAGTACTCAGGATGCTGAAACAGGAGGATTGCCTGAC
TGGGAGATATAAGGAGAATCTGTTGTCACCCCCACCCCTCCCCATAAAGGCAGAATAAAAGAACGTCCTATAAACAAATA
AACAAACAACCCAATAAAACAAAACCAAGATCTCTCCACCTTTTCTTGCTTTTTCAGACTTTGTAATAAGGCCCTTGG
AGTGCAGGATATTCGGCAGGACAAGCAGAGAGGGAGACCATCAGTTCTTTCTTGATCAAGAAGACTATGTTCCTTAGCA
AACTGGTGTGTATTATCTCTTATGCAATGAGCCTGGAAAGAGGGCACAGCCACCGAGGATGGTACAGCATGGATGGATGG
TACGCTACAGAGACTCGGGAGCCCAACTGTGAGTGGCTGACTGGCATGGTAGGTTCAGGGAAGAATTGGCCTGTGAAGAA
AATGTTCTTGAAAAGTGAACAAGGTGCAGGAGGTAGGAGTGGGTCCTGGGCAAAGCAGGGGGTGCATCCCAGCCTCAGGG
AATAGCACAGCAGAGGTCTGTTGATGCATGCGAGTGCATGACCTGCTTGCCAATAGACGATCAAGAATGGGCAAAGCATC
ATGGGTGATGAGTGGGAGAGGGGATGAGACATTCCTTTCTCCCTGCTGAGACTTCCATTGAACCGATGAGTTCTGAATAG
AAGATGCCCCCCCACCCCCCCACCAGTGTAGAATCTGAAGGGAGGCATATATTACCCTATATTACTCTGTGTTGGCGGCG
AGCTATCTGACAGCCAACCTTCCCATACATTTCATTGGGCATACACTAATGACAGGAAGTTCCTTTTGCTTGTATGCAAG
AGATGGCTCACACGATGGAGAATTTAATCTTGTTAGTTTGTTATTTATGTGTCCTAAATTTTGTTCAATAAAAATGAAAC
ACTCCTATG

The examples of SINE B2 embedded in cDNA clones sequence are as following, but is limited to them.

| Gene with AS_refseq | start B2 | end B2 | | strand | subtype | | | | | Legend; cDNA cloneID_alignment Start position_Alignent end_type of Repeat element |
|---|---|---|---|---|---|---|---|---|---|---|
| NM_1771 82 | 973 | 1013 | -2766 | + | B2_M m2 | SINE/ B2 | 2 | 42 | -153 | >AK032380_973_10 13_+_B2_Mm2 |
| NM_0093 51 | 859 | 1044 | -1470 | + | B2_M m1t | SINE/ B2 | 2 | 185 | -8 | >AK033525_859_10 44_+_B2_Mmlt |
| NM_1445 15 | 1564 | 1718 | -761 | + | B2_M m2 | SINE/ B2 | 1 | 158 | -37 | >AK033993_1564_1 718_+_B2_Mm2 |
| NM_1983 00 | 520 | 712 | -1160 | + | B2_M m2 | SINE/ B2 | 1 | 188 | -7 | >AK039361_520_71 2_+_B2_Mm2 |
| NM_0284 28 | 1801 | 1997 | -245 | + | B2_M m1t | SINE/ B2 | 1 | 193 | 0 | >AK042841_1801_1 997_+_B2_Mmlt |
| NM_0106 61 | 946 | 1111 | -1515 | + | B2_M m2 | SINE/ B2 | 1 | 195 | 0 | >AK043817_946_11 11_+_B2_Mm2 |
| NM_0074 85 | 722 | 912 | -417 | + | B2_M m2 | SINE/ B2 | 1 | 194 | -1 | >AK044205_722_91 2_+_B2_Mm2 |
| NM_0106 33 | 971 | 1164 | -67 | + | B2_M m2 | SINE/ B2 | 1 | 195 | 0 | >AK047213_971_11 64_+_B2_Mm2 |
| NM_0302 07 | 714 | 787 | -446 | + | B2_M m2 | SINE/ B2 | 1 | 88 | -107 | >AK079217_714_78 7_+_B2_Mm2 |
| NM_1454 70 | 1498 | 1697 | -1057 | + | B2_M m2 | SINE/ B2 | 1 | 193 | -2 | >AK081722_1498_1 697_+_B2_Mm2 |
| NM_0242 82 | 1379 | 1555 | -2 | + | B2_M m2 | SINE/ B2 | 2 | 187 | -8 | >AK132990_1379_1 555_+_B2_Mm2 |
| NM_1339 94 | 1001 | 1057 | -86 | + | B2_M m2 | SINE/ B2 | 2 | 58 | -137 | >AK133457_1001_1 057_+_B2_Mm2 |
| NM_1339 94 | 1057 | 1137 | -6 | + | B2_M m2 | SINE/ B2 | 91 | 171 | -24 | >AK133457_1057_1 137_+_B2_Mm2 |
| NM_1782 44 | 2323 | 2513 | -45 | + | B2_M m2 | SINE/ B2 | 1 | 195 | 0 | >AK133632_2323_2 513_+_B2_Mm2 |
| NM_0089 97 | 734 | 923 | -3 | + | B2_M m2 | SINE/ B2 | 1 | 186 | -9 | >AK133808_734_92 3_+_B2_Mm2 |
| NM_0805 55 | 286 | 382 | -4 | + | B2_M m1t | SINE/ B2 | 75 | 171 | -22 | >AK134674_286_38 2_+_B2_Mmlt |
| NM_0103 32 | 2428 | 2606 | -1 | + | B2_M m2 | SINE/ B2 | 1 | 183 | -12 | >AK135599_2428_2 606_+_B2_Mm2 |
| NM_1724 67 | 2948 | 3108 | -3 | + | B2_M m2 | SINE/ B2 | 1 | 162 | -33 | >AK137583_2948_3 108_+_B2_Mm2 |
| NM_1751 15 | 81 | 274 | -2006 | + | B2_M m2 | SINE/ B2 | 2 | 195 | 0 | >AK138675_81_274 _+_B2_Mm2 |
| NM_0100 71 | 3150 | 3339 | -54 | + | B2_M m2 | SINE/ B2 | 1 | 195 | 0 | >AK155102_3150_3 339_+_B2_Mm2 |
| NM_1830 14 | 288 | 442 | -20 | + | B3A | SINE/ B2 | 48 | 198 | 0 | >AK015655_288_44 2_+_B3A |
| NM_0265 55 | 277 | 435 | -24 | + | B3A | SINE/ B2 | 40 | 198 | 0 | >AK021299_277_43 5_+_B3A |
| NM_0330 77 | 1109 | 1295 | -1732 | + | B3A | SINE/ B2 | 5 | 189 | -9 | >AK029689_1109_1 295_+_B3A |
| NM_0197 89 | 1052 | 1211 | -1195 | + | B3 | SINE/ B2 | 33 | 196 | -20 | >AK030353_1052_1 211_+_B3 |
| NM_1788 91 | 2403 | 2558 | -3 | + | B3 | SINE/ B2 | 20 | 176 | -40 | >AK030551_2403_2 558_+_B3 |
| NM_1455 79 | 2546 | 2719 | -346 | + | B3 | SINE/ B2 | 2 | 206 | -10 | >AK031007_2546_2 719_+_B3 |
| NM_0085 10 | 2255 | 2404 | -429 | + | B3A | SINE/ B2 | 57 | 198 | 0 | >AK034030_2255_2 404_+_B3A |
| NM_1535 79 | 1071 | 1262 | -798 | + | B3 | SINE/ B2 | 1 | 214 | -2 | >AK035406_1071_1 262_+_B3 |
| NM_1459 42 | 1006 | 1219 | -681 | + | B3 | SINE/ B2 | 2 | 216 | 0 | >AK037188_1006_1 219_+_B3 |
| NM_0257 88 | 1871 | 2045 | -757 | + | B3 | SINE/ B2 | 1 | 215 | -1 | >AK039409_1871_2 045_+_B3 |
| NM_0080 19 | 877 | 1044 | -1057 | + | B3 | SINE/ B2 | 2 | 211 | -5 | >AK040162_877_10 44_+_B3 |
| NM_0010 81475 | 1431 | 1582 | -6 | + | B3 | SINE/ B2 | 69 | 216 | 0 | >AK040401_1431_1 582_+_B3 |
| NM_0010 81475 | 1586 | 1745 | -2 | + | B3 | SINE/ B2 | 3 | 206 | -10 | >AK040401_1586_1 745_+_B3 |
| NM_0074 85 | 722 | 810 | -519 | + | B3 | SINE/ B2 | 1 | 89 | -127 | >AK044205_722_81 0_+_B3 |
| NM_0074 85 | 835 | 912 | -417 | + | B3 | SINE/ B2 | 138 | 215 | -1 | >AK044205_835_91 2_+_B3 |
| NM_1787 94 | 826 | 959 | -986 | + | B3A | SINE/ B2 | 2 | 129 | -69 | >AK045196_826_95 9_+_B3A |
| NM_0089 15 | 737 | 940 | -298 | + | B3 | SINE/ B2 | 8 | 201 | -15 | >AK046652_737_94 0_+_B3 |
| NM_0106 33 | 971 | 1096 | -135 | + | B3 | SINE/ B2 | 1 | 129 | -87 | >AK047213_971_10 96_+_B3 |
| NM_1994 76 | 3267 | 3405 | -1039 | + | B3 | SINE/ B2 | 2 | 149 | -67 | >AK04 7540_3267_3 405_+_B3 |
| NM_1447 95 | 2052 | 2248 | -546 | + | B3 | SINE/ B2 | 2 | 210 | -6 | >AK048854_2052_2 248_+_B3 |
| #N/A | 1858 | 2028 | -5 | + | B3A | SINE/ B2 | 1 | 176 | -22 | >AK049524_1858_2 028_+_B3A |
| NM_0010 38621 | 1207 | 1363 | -359 | + | B3A | SINE/ B2 | 2 | 160 | -38 | >AK053130_1207_1 363 + B3A |
| NM_1814 23 | 1666 | 1733 | -71 | + | B3A | SINE/ B2 | 2 | 72 | -126 | >AK054359_1666_1 733_+_B3A |
| NM_1535 15 | 1403 | 1520 | -470 | + | B3A | SINE/ B2 | 63 | 183 | -15 | >AK078013_1403_1 520_+_B3A |
| NM_1984 15 | 403 | 623 | -1212 | + | B3 | SINE/ B2 | 1 | 216 | 0 | >AK078328_403_62 3_+_B3 |
| NM_1522 20 | 1794 | 1979 | -561 | + | B3 | SINE/ B2 | 3 | 213 | -3 | >AK078537_1794_1 979_+_B3 |
| NM_0257 29 | 1 | 165 | -203 | + | B3 | SINE/ B2 | 15 | 177 | -39 | >AK079403_1_165_ +_B3 |
| NM_0166 93 | 1307 | 1517 | -13 | + | B3 | SINE/ B2 | 2 | 216 | 0 | >AK080235_1307_1 517_+_B3 |
| NM_0101 51 | 814 | 1024 | -451 | + | B3 | SINE/ B2 | 2 | 216 | 0 | >AK082108_814_10 24_+_B3 |
| NM_0097 13 | 1690 | 1882 | -5 | + | B3 | SINE/ B2 | 1 | 199 | -17 | >AK082325_1690_1 882_+_B3 |
| NM_0010 29985 | 221 | 280 | -1179 | + | B3 | SINE/ B2 | 108 | 168 | -48 | >AK084376_221_28 0_+_B3 |
| NM_0097 37 | 1805 | 2005 | -406 | + | B3 | SINE/ B2 | 1 | 207 | -9 | >AK085337_1805_2 005_+_B3 |
| NM_0270 81 | 984 | 1130 | -1155 | + | B3 | SINE/ B2 | 1 | 162 | -54 | >AK086470_984_11 30_+_B3 |
| NM_0187 79 | 876 | 1062 | -530 | + | B3A | SINE/ B2 | 2 | 188 | -10 | >AK089148_876_10 62_+_B3A |
| NM_0279 19 | 1468 | 1667 | -185 | + | B3 | SINE/ B2 | 1 | 213 | -3 | >AK090182_1468_1 667_+_B3 |
| NM_0110 34 | 434 | 608 | -12 | + | B3 | SINE/ B2 | 2 | 216 | 0 | >AK131819 434 60 8_+_B3 |
| NM_1755 51 | 1802 | 1895 | -513 | + | B3A | SINE/ B2 | 64 | 176 | -22 | >AK132737_1802_1 895_+_B3A |
| NM_1755 51 | 1829 | 1928 | -480 | + | B3 | SINE/ B2 | 101 | 213 | -3 | >AK132737_1829_1 928_+_B3 |
| NM_0079 21 | 951 | 1134 | -1650 | + | B3A | SINE/ B2 | 3 | 187 | -11 | >AK133325_951_11 34_+_B3A |
| NM_0010 39042 | 1220 | 1315 | -292 | + | B3A | SINE/ B2 | 48 | 142 | -56 | >AK134755_1220_1 315_+_B3A |
| NM_1773 28 | 1378 | 1504 | -285 | + | B3 | SINE/ B2 | 85 | 216 | 0 | >AK134874_1378_1 504_+_B3 |
| NM_0218 99 | 1172 | 1259 | -140 | + | B3 | SINE/ B2 | 1 | 95 | -121 | >AK135206_1172_1 259_+_B3 |
| NM_0087 05 | 1521 | 1717 | -572 | + | B3A | SINE/ B2 | 1 | 197 | -1 | >AK136279_1521_1 717_+_B3A |
| NM_0010 79932 | 2116 | 2299 | -702 | + | B3 | SINE/ B2 | 6 | 208 | -8 | >AK137643_2116_2 299_+_B3 |
| NM_0010 79932 | 3059 | 3253 | -2 | + | B3 | SINE/ B2 | 4 | 205 | -11 | >AK137643_3059_3 253_+ _B3 |
| NM_0010 33286 | 2524 | 2698 | -63 | + | B3A | SINE/ B2 | 4 | 181 | -17 | >AK138296_2524_2 698_+_B3A |
| NM_0089 62 | 928 | 1095 | -375 | + | B3 | SINE/ B2 | 1 | 153 | -63 | >AK138521_928_10 95_+_B3 |
| NM_1753 49 | 2151 | 2347 | -181 | + | B3A | SINE/ B2 | 11 | 196 | -2 | >AK139254_2151_2 347_+_B3A |
| NM_1770 03 | 1277 | 1466 | 0 | + | B3 | SINE/ B2 | 1 | 200 | -16 | >AK139647_1277_1 466_+_B3 |
| NM_0079 65 | 1338 | 1488 | -5 | + | B3 | SINE/ B2 | 51 | 216 | 0 | >AK140072_1338_1 488_+_B3 |
| NM_0101 92 | 1808 | 1933 | -45 | + | B3 | SINE/ B2 | 1 | 126 | -90 | >AK140346_1808_1 933_+_B3 |
| NM_0274 46 | 1871 | 1983 | -2794 | + | B3A | SINE/ B2 | 1 | 130 | -68 | >AK140616_1871_1 983_+_B3A |
| NM_0274 46 | 2318 | 2342 | -2435 | + | B3A | SINE/ B2 | 131 | 156 | -42 | >AK140616_2318_2 342_+_B3A |
| NM_1771 86 | 2061 | 2142 | -351 | + | B3 | SINE/ B2 | 130 | 211 | -5 | >AK142359_2061_2 142_+_B3 |
| NM_1451 34 | 2963 | 3148 | -1292 | + | B3 | SINE/ B2 | 28 | 210 | -6 | >AK142507_2963_3 148_+_B3 |
| NM_0098 90 | 2740 | 2968 | -135 | + | B3 | SINE/ B2 | 1 | 216 | 0 | >AK142879_2740_2 968_+_B3 |
| NM_1460 55 | 5202 | 5272 | -94 | + | B3 | SINE/ B2 | 60 | 141 | -75 | >AK143143_5202_5 272_+B3 |
| NM_0089 77 | 1344 | 1513 | -645 | + | B3 | SINE/ B2 | 11 | 205 | -11 | >AK143279_1344_1 513_+_B3 |
| NM_0260 36 | 2001 | 2167 | -272 | + | B3A | SINE/ B2 | 1 | 169 | -29 | >AK149843_2001_2 167_+_B3A |
| NM_0011 59519 | 1440 | 1548 | -1422 | + | B3A | SINE/ B2 | 55 | 163 | -35 | >AK157402_1440_1 548_+_B3A |
| NM_0011 59519 | 2503 | 2556 | -558 | + | B3 | SINE/ B2 | 1 | 56 | -160 | >AK157402_2503_2 556_+_B3 |
| NM_0011 59519 | 2544 | 2616 | -498 | + | B3 | SINE/ B2 | 109 | 177 | -39 | >AK157402 2544 2 616_+_B3 |
| NM_0011 59519 | 2702 | 2896 | -412 | + | B3A | SINE/ B2 | 1 | 198 | 0 | >AK157402_2702_2 896_+_B3A |
| NM_0011 10504 | 1778 | 1909 | -25 | + | B3 | SINE/ B2 | 58 | 210 | -6 | >AK160921_1778_1 909_+_B3 |
| NM_0307 14 | 2153 | 2335 | -62 | + | B3 | SINE/ B2 | 40 | 216 | 0 | >AK165234_2153_2 335_+_B3 |
| NM_0258 25 | 945 | 1052 | -383 | R C | B2_M mla | SINE/ B2 | -85 | 108 | 1 | >AK014613_945_10 52_RC_B2_Mmla |
| NM_1337 56 | 1102 | 1289 | -246 | R C | B2_M m2 | SINE/ B2 | -4 | 191 | 1 | >AK016234_1102_1 289_RC_B2_Mm2 |
| NM_1832 94 | 1565 | 1750 | -1384 | R C | B2_M mla | SINE/ B2 | -8 | 185 | 4 | >AK029702_1565_1 750_RC_B2_Mmla |
| NM_0094 46 | 531 | 710 | -2730 | R C | B2_M m2 | SINE/ B2 | -6 | 189 | 2 | >AK030803_531_71 0_RC_B2_Mm2 |
| NM_0257 88 | 1687 | 1839 | -963 | R C | B2_M m2 | SINE/ B2 | -11 | 184 | 1 | >AK039409_1687_1 839_RC_B2_Mm2 |
| NM_1777 85 | 1626 | 1682 | -1130 | R C | B2_M m2 | SINE/ B2 | -4 | 191 | 137 | >AK040275_1626_1 682_RC_B2_Mm2 |
| NM_1777 85 | 1682 | 1739 | -1073 | R C | B2_M m2 | SINE/ B2 | -94 | 101 | 46 | >AK040275_1682_1 739 _RC_B2_Mm2 |
| NM_1777 85 | 1776 | 1952 | -1036 | R C | B2_M mlt | SINE/ B2 | -13 | 180 | 2 | >AK040275_1776_1 952_RC_B2_Mmlt |
| NM_0074 85 | 1137 | 1341 | -192 | R C | B2_M m2 | SINE/ B2 | 0 | 195 | 2 | >AK044205_1137_1 341_RC_B2_Mm2 |
| NM_1752 73 | 537 | 714 | -1793 | R C | B2_M m2 | SINE/ B2 | -5 | 190 | 1 | >AK048762_537_71 4_RC_B2_Mm2 |
| NM_0294 09 | 1005 | 1172 | -2204 | R C | B2_M mlt | SINE/ B2 | -14 | 179 | 1 | >AK049449_1005_1 172_RC_B2 Mmlt |
| NM_0078 36 | 282 | 407 | -2067 | R C | B2_M m2 | SINE/ B2 | -67 | 128 | 2 | >AK054076_282_40 7 _RC_B2_Mm2 |
| NM_0078 36 | 1764 | 1860 | -614 | R C | B2_M m2 | SINE/ B2 | -97 | 98 | 2 | >AK054076_1764_1 860_RC_B2_Mm2 |
| NR_0028 91 | 1 | 181 | -2344 | R C | B2_M mla | SINE/ B2 | -10 | 183 | 2 | >AK076350_1_181_ RC_B2_Mmla |
| NM_0265 00 | 984 | 1157 | -1501 | R C | B2_M m2 | SINE/ B2 | -14 | 181 | 1 | >AK076438_984_11 57_RC_B2_Mm2 |
| NM_0529 94 | 2158 | 2323 | -1225 | R C | B2_M m2 | SINE/ B2 | -4 | 191 | 8 | >AK079094_2158_2 323_RC_B2_Mm2 |
| NM_1531 00 | 1870 | 2057 | -1774 | R C | B2_M m2 | SINE/ B2 | 0 | 195 | 1 | >AK086953_1870_2 057_RCB2_Mm2 |
| NM_1753 13 | 2088 | 2261 | -2958 | R C | B2_M m2 | SINE/ B2 | -9 | 186 | 14 | >AK132441_2088_2 261_RC_B2_Mm2 |
| NM_0011 14140 | 5305 | 5461 | -1145 | R C | B2_M m2 | SINE/ B2 | -38 | 157 | 2 | >AK133162_5305_5 461_RC_B2_Mm2 |
| NM_0095 79 | 1059 | 1251 | -2941 | R C | B2_M m2 | SINE/ B2 | 0 | 195 | 1 | >AK137370_1059_1 251_RC_B2_Mm2 |
| NM_0105 67 | 1613 | 1794 | -1882 | R C | B2_M m2 | SINE/ B2 | -2 | 193 | 1 | >AK138181_1613_1 794_RC_B2_Mm2 |
| NM_0010 08423 | 2088 | 2222 | -57 | R C | B2_M m2 | SINE/ B2 | -60 | 135 | 1 | >AK141165_2088_2 222_RC_B2_Mm2 |
| NM_1990 27 | 935 | 1064 | -63 | R C | B2_M m2 | SINE/ B2 | -65 | 130 | 1 | >AK141411_935_10 64_RC_B2_Mm2 |
| NM_0010 33316 | 3103 | 3283 | -440 | R C | B2_M m2 | SINE/ B2 | -5 | 190 | 2 | >AK145736_3103_3 283_RC_B2_Mm2 |
| NR_0034 92 | 748 | 810 | -977 | R C | B2_M mla | SINE/ B2 | -6 | 187 | 123 | >AK147092_748_81 0_RC_B2_Mmla |
| NM_0105 67 | 935 | 1129 | -78 | R C | B2_M m2 | SINE/ B2 | 0 | 195 | 2 | >AK148373_935_11 29_RC_B2_Mm2 |
| NM_0261 15 | 3593 | 3791 | -204 | R C | B2_M m2 | SINE/ B2 | -1 | 194 | 1 | >AK155374_3593_3 791_RC_B2_Mm2 |
| NM_0103 98 | 1080 | 1265 | -909 | R C | B2_M m2 | SINE/ B2 | -8 | 187 | 1 | >AK157261_1080_1 265_RC_B2_Mm2 |
| NM_0078 93 | 764 | 820 | -1012 | R C | B2_M m2 | SINE/ B2 | -94 | 101 | 47 | >AK163105_764_82 0_RC_B2_Mm2 |
| NM_0078 93 | 894 | 1062 | -938 | R C | B2_M m2 | SINE/ B2 | -13 | 182 | 1 | >AK163105_894_10 62_RC_B2_Mm2 |
| NM_0078 93 | 1390 | 1436 | -564 | R C | B2_M m2 | SINE/ B2 | -5 | 190 | 137 | >AK163105_1390_1 436_RC_B2_Mm2 |
| NM_0078 93 | 1436 | 1490 | -510 | R C | B2_M m2 | SINE/ B2 | -94 | 101 | 47 | >AK163105_1436_1 490_RC_B2_Mm2 |
| NM_0078 93 | 1543 | 1701 | -299 | R C | B2_M mlt | SINE/ B2 | -24 | 169 | 1 | >AK163105_1543_1 701_RC_B2_Mmlt |
| NM_1771 86 | 1783 | 1964 | -258 | R C | B2_M mlt | SINE/ B2 | -7 | 186 | 2 | >AK163831_1783_1 964_RC_B2_Mmlt |
| NM_0307 14 | 1401 | 1588 | -809 | R C | B2_M m2 | SINE/ B2 | 0 | 195 | 3 | >AK165234_1401_1 588_RC_B2_Mm2 |
| NM_0011 10101 | 1253 | 1420 | -1139 | R C | B2_M m2 | SINE/ B2 | -22 | 173 | 2 | >AK169421_1253_1 420_RC_B2_Mm2 |
| NM_0076 01 | 387 | 544 | -1715 | R C | B3A | SINE/ B2 | -22 | 176 | 1 | >AK016423_387_54 4_RC_B3A |
| NM_0273 46 | 1459 | 1690 | -74 | R C | B3 | SINE/ B2 | -2 | 214 | 1 | >AK019925_1459_1 690_RC_B3 |
| NM_1386 64 | 1382 | 1411 | -1334 | R C | B3A | SINE/ B2 | -14 8 | 50 | 21 | >AK028982_1382_1 411_RC_B3A |
| NM_0138 40 | 160 | 290 | -556 | R C | B3 | SINE/ B2 | -79 | 137 | 9 | >AK029359_160_29 0_RC_B3 |
| NM_0138 40 | 774 | 960 | -72 | R C | B3 | SINE/ B2 | -7 | 209 | 1 | >AK029359_774_96 0_RC_B3 |
| NM_1535 91 | 769 | 982 | -1569 | R C | B3 | SINE/ B2 | -6 | 210 | 1 | >AK032194_769_98 2_RC_B3 |
| NM_0287 94 | 1926 | 2130 | -654 | R C | B3 | SINE/ B2 | -12 | 204 | 2 | >AK032215_1926_2 130_RC_B3 |
| NM_0010 12311 | 1317 | 1437 | -1013 | R C | B3 | SINE/ B2 | -44 | 172 | 38 | >AK034331_1317_1 437_RC_B3 |
| NM_0010 12311 | 1335 | 1437 | -1013 | R C | B3A | SINE/ B2 | -54 | 144 | 38 | >AK034331_1335_1 437_RC_B3A |
| NM_1341 22 | 1094 | 1205 | -719 | R C | B3 | SINE/ B2 | -88 | 128 | 1 | >AK035015_1094_1 205_RC_B3 |
| NM_1535 79 | 1443 | 1617 | -577 | R C | B3 | SINE/ B2 | -2 | 214 | 45 | >AK035406_1443_1 617_RC_B3 |
| NM_1535 79 | 1483 | 1633 | -561 | R C | B3A | SINE/ B2 | -18 | 180 | 27 | >AK035406_1483_1 633_RC_B3A |
| NM_0010 81014 | 1615 | 1715 | -218 | R C | B3A | SINE/ B2 | -88 | 110 | 1 | >AK039704_1615_1 715_RC_B3A |
| NM_0187 47 | 432 | 531 | -1672 | R C | B3A | SINE/ B2 | -31 | 167 | 49 | >AK040672_432_53 1_RC_B3A |
| NM_1768 41 | 1677 | 1886 | -64 | R C | B3 | SINE/ B2 | 0 | 216 | 2 | >AK041236_1677_1 886_RC_B3 |
| NM_1338 78 | 1676 | 1882 | -314 | R C | B3 | SINE/ B2 | -7 | 209 | 1 | >AK041654_1676_1 882_RC_B3 |
| NM_1452 15 | 2334 | 2533 | -484 | R C | B3 | SINE/ B2 | 0 | 216 | 3 | >AK041742_2334_2 533_RC_B3 |
| NM_1453 69 | 1132 | 1336 | -593 | R C | B3 | SINE/ B2 | -7 | 209 | 5 | >AK042861_1132_1 336_RC_B3 |
| NM_1726 91 | 248 | 442 | -151 | R C | B3A | SINE/ B2 | -18 | 180 | 10 | >AK043958_248_44 2_RC_B3A |
| NM_0074 85 | 938 | 1127 | -202 | R C | B3 | SINE/ B2 | -37 | 179 | 2 | >AK044205_938_11 27_RC_B3 |
| NM_0074 85 | 989 | 1127 | -202 | R C | B3A | SINE/ B2 | -57 | 141 | 2 | >AK044205_989_11 27_RC_B3A |
| NM_0079 25 | 592 | 713 | -719 | R C | B3 | SINE/ B2 | -54 | 162 | 39 | >AK045677_592_71 3_RC_B3 |
| NM_1770 06 | 3566 | 3620 | -1104 | R C | B3 | SINE/ B2 | -16 0 | 56 | 2 | >AK046828_3566_3 620_RC_B3 |
| NM_0106 33 | 431 | 640 | -591 | R C | B3 | SINE/ B2 | -4 | 212 | 8 | >AK047213_431_64 0_RC_B3 |
| NM_0106 33 | 457 | 640 | -591 | R C | B3A | SINE/ B2 | -7 | 191 | 8 | >AK047213_457_64 0_RC_B3A |
| NM_0088 42 | 1460 | 1636 | -53 | R C | B3A | SINE/ B2 | -4 | 194 | 10 | >AK047301_1460_1 636_RC_B3A |
| NM_1994 76 | 2716 | 2780 | -1664 | R C | B3A | SINE/ B2 | -36 | 162 | 98 | >AK047540_2716_2 780_RC_B3A |
| NM_0135 14 | 1203 | 1416 | -311 | R C | B3 | SINE/ B2 | 0 | 216 | 1 | >AK048309_1203_1 416_RC_B3 |
| NM_0807 93 | 2342 | 2396 | -1333 | R C | B3A | SINE/ B2 | -5 | 193 | 144 | >AK048747_2342_2 396_RC_B3A |
| NM_0807 93 | 2430 | 2530 | -1199 | R C | B3A | SINE/ B2 | -55 | 143 | 47 | >AK048747_2430_2 530_RC_B3A |
| NM_0010 38621 | 159 | 275 | -1447 | R C | B3 | SINE/ B2 | -56 | 160 | 34 | >AK053130_159_27 5_RC_B3 |
| NM_0078 36 | 1747 | 1860 | -614 | R C | B3 | SINE/ B2 | -97 | 119 | 2 | >AK054076_1747_1 860_RC_B3 |
| NM_0108 78 | 1134 | 1337 | -1192 | R C | B3 | SINE/ B2 | 0 | 216 | 1 | >AK078161_1134_1 337_RC_B3 |
| NM_0116 70 | 521 | 690 | -995 | R C | B3 | SINE/ B2 | -60 | 156 | 1 | >AK078321_521_69 0_RC_B3 |
| NM_0260 86 | 882 | 1046 | -355 | R C | B3A | SINE/ B2 | -18 | 180 | 1 | >AK079515_882_10 46_RC_B3A |
| NM_0253 96 | 1081 | 1179 | -370 | R C | B3 | SINE/ B2 | -27 | 189 | 75 | >AK080749_1081_1 179_RC_B3 |
| NM_0079 23 | 2420 | 2567 | -582 | R C | B3A | SINE/ B2 | -48 | 150 | 4 | >AK086589_2420_2 567_RC_B3A |
| NM_0284 27 | 1420 | 1597 | -495 | R C | B3 | SINE/ B2 | -5 | 211 | 2 | >AK090347_1420_1 597_RC_B3 |
| NM_0261 57 | 1871 | 2066 | -1227 | R C | B3A | SINE/ B2 | 0 | 198 | 5 | >AK132393_1871_2 066_RC_B3A |
| NM_0261 57 | 2342 | 2408 | -885 | R C | B3A | SINE/ B2 | -13 1 | 67 | 1 | >AK132393_2342_2 408_RC_B3A |
| NM_0261 57 | 2473 | 2606 | -687 | R C | B3A | SINE/ B2 | -25 | 173 | 28 | >AK132393_2473_2 606_RC_B3A |
| NM_1753 13 | 2113 | 2261 | -2958 | R C | B3 | SINE/ B2 | -40 | 176 | 14 | >AK132441_2113_2 261_RC_B3 |
| NM_1753 13 | 4127 | 4308 | -948 | R C | B3 | SINE/ B2 | -6 | 210 | 28 | >AK132441_4127_4 308_RC_B3 |
| NM_0242 82 | 1137 | 1290 | -91 | R C | B3A | SINE/ B2 | -39 | 159 | 1 | >AK132990_1137_1 290_RC_B3A |
| NM_1773 28 | 277 | 358 | -1283 | R C | B3A | SINE/ B2 | -56 | 142 | 66 | >AK134874_277_35 8_RC_B3A |
| NM_1773 28 | 405 | 484 | -1157 | R C | B3A | SINE/ B2 | -13 3 | 65 | 1 | >AK134874_405_48 4_RC_B3A |
| NM_1773 28 | 545 | 713 | -928 | R C | B3A | SINE/ B2 | -4 | 194 | 14 | >AK134874_545_71 3_RC_B3A |
| NM_1773 28 | 787 | 971 | -670 | R C | B3A | SINE/ B2 | -4 | 194 | 7 | >AK134874_787_97 1_RC_B3A |
| NM_0218 99 | 161 | 307 | -1092 | R C | B3A | SINE/ B2 | -46 | 152 | 1 | >AK135206_161_30 7_RC_B3A |
| NM_0103 32 | 228 | 409 | -1967 | R C | B3 | SINE/ B2 | -28 | 188 | 2 | >AK135599_228_40 9_RC_B3 |
| NM_1724 07 | 1470 | 1683 | -74 | R C | B3 | SINE/ B2 | -5 | 211 | 2 | >AK143014_1470_1 683_RC_B3 |
| NM_0010 09935 | 1389 | 1495 | -157 | R C | B3 | SINE/ B2 | -96 | 120 | 1 | >AK143784_1389_1 495_RC_B3 |
| NM_0198 27 | 2953 | 3143 | -1064 | R C | B3 | SINE/ B2 | -1 | 215 | 1 | >AK145079_2953_3 143_RC_B3 |
| NM_0112 12 | 2131 | 2283 | -1263 | R C | B3A | SINE/ B2 | -47 | 151 | 2 | >AK148045_2131_2 283_RC_B3A |
| NM_0105 67 | 984 | 1129 | -78 | R C | B3A | SINE/ B2 | -60 | 138 | 2 | >AK148373_984_11 29_RC_B3A |
| NM_0011 10504 | 1406 | 1594 | -400 | R C | B3A | SINE/ B2 | -10 | 188 | 2 | >AK149403_1406_1 594_RC_B3A |
| NM_0260 36 | 2270 | 2439 | 0 | R C | B3 | SINE/ B2 | -4 | 212 | 27 | >AK149843_2270_2 439_RC_B3 |
| NM_0103 98 | 1292 | 1476 | -698 | R C | B3A | SINE/ B2 | -11 | 187 | 3 | >AK157261_1292_1 476_RC_B3A |
| NM_0011 10504 | 1292 | 1362 | -572 | R C | B3A | SINE/ B2 | -11 2 | 86 | 11 | >AK160921_1292_1 362_RC_B3A |
| NM_0078 93 | 1080 | 1254 | -746 | R C | B3 | SINE/ B2 | 0 | 216 | 21 | >AK163105_1080_1 254_RC_B3 |
| NM_0078 93 | 1427 | 1491 | -509 | R C | B3A | SINE/ B2 | -82 | 116 | 46 | >AK163105_1427_1 491_RC_B3A |
| NM_0307 14 | 1954 | 2064 | -333 | R C | B3 | SINE/ B2 | -95 | 121 | 2 | >AK165234_1954_2 064_RC_B3 |
| NM_0011 10101 | 853 | 1043 | -1516 | R C | B3 | SINE/ B2 | -5 | 211 | 6 | >AK169421_853_10 43_RC_B3 |
| NM_0011 10101 | 1742 | 1936 | -623 | R C | B3 | SINE/ B2 | -2 | 214 | 8 | >AK169421_1742_1 936_RC_B3 |

## Claims

1. A functional nucleic acid molecule, comprising:
(a) a target determinant sequence comprising antisense sequence to a target sequence in the protein-encoding RNA for which protein synthesis efficiency is to be increased; and
(b) a regulatory sequence having an activity of increasing of the protein synthesis efficiency,
wherein the regulatory sequence comprises a SINE (Short Interspersed Element) B2 derived sequence with at least 60% similarity to the RNA which is encoded by a DNA consisting of SEQ ID NO: 1, 2 or 3.

2. The functional nucleic acid molecule as set forth in claim 1, wherein the target determinant sequence is located between a 5'-terminal and the regulatory sequence in the synthetic functional RNA molecule.

3. The functional nucleic acid molecule as set forth in claim 1 or claim 2, wherein the target determinant sequence has a length of 7 nucleotides to 250 nucleotides.

4. The functional nucleic acid molecule as set forth in claim any one of claims 1 to 3, wherein the target determinant sequence has at least 60% similarity to a sequence complementary to the target sequence to a corresponding sequence in the protein-encoding RNA or a sequence around the first 5'-terminal start codon of the protein-encoding sequence.

5. The functional nucleic acid molecule as set forth in any one of claims 1 to 4, wherein the regulatory sequence of the functional nucleic acid molecule is oriented in a reverse direction relative to the direction of translation.

6. The functional nucleic acid molecule as set forth in any one of claims 1 to 5, wherein the target determinant sequence is designed to be hybridizable with a 5'-UTR (untranslated region) of the protein-encoding RNA or a sequence around the first 5'-terminal start codon of the protein-encoding RNA.

7. An *in vitro* method for producing a functional nucleic acid molecule, comprising the step of preparing an RNA molecule that comprises:
(a) a target determinant sequence comprising antisense sequence to a target sequence in the protein-encoding RNA for which protein synthesis efficiency is to be increased; and
(b) a regulatory sequence having an activity of increasing of the protein synthesis efficiency,
wherein the regulatory sequence comprises a SINE (Short Interspersed Element) B2 derived sequence with at least 60% similarity to the RNA which is encoded by a DNA consisting of SEQ ID NO: 1, 2 or 3.

8. A DNA molecule encoding a functional nucleic acid molecule as set forth in any one of claims 1 to 6.

9. An expression vector comprising a functional nucleic acid molecule as set forth in any one of claims 1 to 6 or a DNA molecule that encodes as set forth in claim 8.

10. A composition for increasing protein synthesis efficiency which comprises a functional nucleic acid molecule as set forth in any one of claims 1 to 6, a DNA molecule as set forth in claim 8, or an expression vector as set forth in claim 9.

11. An *in vitro* method for increasing the protein synthesis efficiency, comprising the step of:
(a) allowing a functional nucleic acid molecule as set forth in any one of claims 1 to 6 to coexist with a protein-encoding RNA which partial sequence of the protein-encoding RNA has similarity with the target determinant sequence of the functional nucleic acid molecule.

12. The method for increasing the protein synthesis efficiency as set forth in claim 11, wherein:
the step (a) comprises transfecting into a cell the functional nucleic acid molecule as set forth in any one of claims 1 to 6 or a DNA molecule as set forth in claim 8.

13. An *in vitro* method for producing a protein, comprising the step of increasing protein synthesis efficiency by a method for increasing the protein synthesis efficiency as set forth in claim 11 or 12.

14. The functional nucleic acid molecule as set forth in any one of claims 1 to 6, the DNA molecule as set forth in claim 8, or the expression vector as set forth in claim 9, for use in medicine.

15. The functional nucleic acid molecule as set forth in any one of claims 1 to 6, the DNA molecule as set forth in claim 8, or the expression vector as set forth in claim 9, for use in the treatment of a haploinsufficiency or a disease that is caused by a quantitative decrease in a protein.

## Patentansprüche

1. Funktionelles Nukleinsäuremolekül, umfassend:
(a) eine Zieldeterminantensequenz, die eine Antisense-Sequenz zu einer Zielsequenz in der proteinkodierenden RNA umfasst, für die die Effizienz der Proteinsynthese erhöht werden soll; und
(b) eine regulatorische Sequenz, die eine Aktivität der Steigerung der Effizienz der Proteinsynthese aufweist,
wobei die regulatorische Sequenz eine von SINE (Short Interspersed Element) B2 abgeleitete Sequenz mit mindestens 60 % Ähnlichkeit zu der RNA umfasst, die von einer DNA kodiert wird, die aus SEQ ID NO: 1, 2 oder 3 besteht.

2. Funktionelles Nukleinsäuremolekül nach Anspruch 1, wobei die Zieldeterminantensequenz zwischen einem 5'-Ende und der regulatorischen Sequenz im synthetischen funktionellen RNA-Molekül liegt.

3. Funktionelles Nukleinsäuremolekül nach Anspruch 1 oder Anspruch 2, wobei die Zieldeterminantensequenz eine Länge von 7 bis 250 Nukleotiden aufweist.

4. Funktionelles Nukleinsäuremolekül nach einem beliebigen der Ansprüche 1 bis 3, wobei die Zieldeterminantensequenz mindestens 60 % Ähnlichkeit mit einer zur Zielsequenz komplementären Sequenz, einer entsprechenden Sequenz in der proteinkodierenden RNA oder einer Sequenz um das erste 5'-Ende-Startcodon der proteinkodierenden Sequenz aufweist.

5. Funktionelles Nukleinsäuremolekül nach einem beliebigen der Ansprüche 1 bis 4, wobei die regulatorische Sequenz des funktionellen Nukleinsäuremoleküls in umgekehrter Richtung zur Translationsrichtung ausgerichtet ist.

6. Funktionelles Nukleinsäuremolekül nach einem beliebigen der Ansprüche 1 bis 5, wobei die Zieldeterminantensequenz dazu ausgelegt ist, mit einer 5'-UTR (untranslatierten Region) der proteinkodierenden RNA oder einer Sequenz um das erste 5'-Ende-Startcodon der proteinkodierenden RNA hybridisierbar zu sein.

7. In-vitro-Verfahren zur Herstellung eines funktionellen Nukleinsäuremoleküls, umfassend den Schritt der Vorbereitung eines RNA-Moleküls, das Folgendes umfasst:
(a) eine Zieldeterminantensequenz, die eine Antisense-Sequenz zu einer Zielsequenz in der proteinkodierenden RNA umfasst, für die die Effizienz der Proteinsynthese erhöht werden soll; und
(b) eine regulatorische Sequenz, die eine Aktivität der Steigerung der Effizienz der Proteinsynthese aufweist,
wobei die regulatorische Sequenz eine von SINE (Short Interspersed Element) B2 abgeleitete Sequenz mit mindestens 60 % Ähnlichkeit zu der RNA umfasst, die von einer DNA kodiert wird, die aus SEQ ID NO: 1, 2 oder 3 besteht.

8. DNA-Molekül, das ein funktionelles Nukleinsäuremolekül nach einem beliebigen der Ansprüche 1 bis 6 kodiert.

9. Expressionsvektor, umfassend ein funktionelles Nukleinsäuremolekül nach einem beliebigen der Ansprüche 1 bis 6 oder ein DNA-Molekül, das nach Anspruch 8 codiert.

10. Zusammensetzung zur Steigerung der Effizienz der Proteinsynthese, die ein funktionelles Nukleinsäuremolekül nach einem beliebigen der Ansprüche 1 bis 6, ein DNA-Molekül nach Anspruch 8 oder einen Expressionsvektor nach Anspruch 9 umfasst.

11. *In-vitro*-Verfahren zur Steigerung der Effizienz der Proteinsynthese, umfassend den folgenden Schritt:
(a) Gestatten des gleichzeitigen Vorliegens eines funktionellen Nukleinsäuremoleküls nach einem beliebigen der Ansprüche 1 bis 6 und einer proteinkodierenden RNA, wobei die Teilsequenz der proteinkodierenden RNA Ähnlichkeit mit der Zieldeterminantensequenz des funktionellen Nukleinsäuremoleküls aufweist.

12. Verfahren zur Steigerung der Effizienz der Proteinsynthese nach Anspruch 11, wobei:
Schritt (a) das Transfektieren des funktionellen Nukleinsäuremoleküls in eine Zelle nach einem beliebigen der Ansprüche 1 bis 6 oder eines DNA-Moleküls nach Anspruch 8 umfasst.

13. In-vitro-Verfahren zur Herstellung eines Proteins, umfassend den Schritt der Steigerung der Effizienz der Proteinsynthese durch ein Verfahren zur Steigerung der Effizienz der Proteinsynthese nach Anspruch 11 oder 12.

14. Funktionelles Nukleinsäuremolekül nach einem beliebigen der Ansprüche 1 bis 6, DNA-Molekül nach Anspruch 8 oder Expressionsvektor nach Anspruch 9 zur Verwendung in der Medizin.

15. Funktionelles Nukleinsäuremolekül nach einem beliebigen der Ansprüche 1 bis 6, DNA-Molekül nach Anspruch 8 oder Expressionsvektor nach Anspruch 9, zur Verwendung bei der Behandlung einer Haploinsuffizienz oder einer Erkrankung, die durch eine quantitative Abnahme eines Proteins verursacht wird.

## Revendications

1. Une molécule d'acide nucléique fonctionnelle, comprenant :
(a) une séquence déterminante cible comprenant une séquence antisens dirigée contre une séquence cible au sein de l'ARN codant pour des protéines, pour laquelle on souhaite accroître l'efficacité de la synthèse protéique ; et
(b) une séquence régulatrice ayant pour activité d'accroître l'efficacité de la synthèse protéique,
dans laquelle la séquence régulatrice comprend une séquence dérivée d'un élément SINE (Short Interspersed Element) de type B2, présentant une similarité d'au moins 60 % avec l'ARN codé par un ADN constitué par la SEQ ID NO : 1, 2 ou 3.

2. Molécule d'acide nucléique fonctionnelle selon la revendication 1, dans laquelle la séquence déterminante cible est située entre une extrémité 5' et la séquence régulatrice dans la molécule d'ARN fonctionnelle synthétique.

3. Molécule d'acide nucléique fonctionnelle selon la revendication 1 ou la revendication 2, dans laquelle la séquence déterminante cible présente une longueur comprise entre 7 et 250 nucléotides.

4. Molécule d'acide nucléique fonctionnelle selon l'une quelconque des revendications 1 à 3, dans laquelle la séquence déterminante cible présente une similarité d'au moins 60 % avec une séquence complémentaire de la séquence cible, avec une séquence correspondante située dans l'ARN codant pour des protéines ou avec une séquence entourant le premier codon d'initiation à l'extrémité 5' de la séquence codant pour des protéines.

5. Molécule d'acide nucléique fonctionnelle selon l'une quelconque des revendications 1 à 4, dans laquelle la séquence régulatrice de la molécule d'acide nucléique fonctionnelle est orientée dans le sens inverse par rapport au sens de la traduction.

6. Molécule d'acide nucléique fonctionnelle selon l'une quelconque des revendications 1 à 5, dans laquelle la séquence déterminante cible est conçue pour pouvoir s'hybrider avec une région 5'-UTR (région non traduite) de l'ARN codant pour des protéines, ou avec une séquence située à proximité du premier codon d'initiation à l'extrémité 5' de l'ARN codant pour des protéines.

7. Procédé *in vitro* de production d'une molécule d'acide nucléique fonctionnelle, comprenant l'étape de préparation d'une molécule d'ARN qui comprend :
(a) une séquence déterminante cible comprenant une séquence antisens dirigée contre une séquence cible au sein de l'ARN codant pour des protéines, pour laquelle on souhaite accroître l'efficacité de la synthèse protéique ; et
(b) une séquence régulatrice ayant pour activité d'accroître l'efficacité de la synthèse protéique,
dans laquelle la séquence régulatrice comprend une séquence dérivée d'un élément SINE (Short Interspersed Element) de type B2, présentant une similarité d'au moins 60 % avec l'ARN codé par un ADN constitué par la SEQ ID NO : 1, 2 ou 3.

8. Molécule d'ADN codant pour une molécule d'acide nucléique fonctionnelle telle que définie dans l'une quelconque des revendications 1 à 6.

9. Vecteur d'expression comprenant une molécule d'acide nucléique fonctionnelle telle que définie dans l'une quelconque des revendications 1 à 6, ou une molécule d'ADN codant, telle que définie dans la revendication 8.

10. Composition destinée à accroître l'efficacité de la synthèse protéique, comprenant une molécule d'acide nucléique fonctionnelle telle que définie dans l'une quelconque des revendications 1 à 6, une molécule d'ADN telle que définie dans la revendication 8, ou un vecteur d'expression tel que défini dans la revendication 9.

11. Procédé *in vitro* pour accroître l'efficacité de la synthèse protéique, comprenant l'étape consistant à :
(a) faire coexister une molécule d'acide nucléique fonctionnelle, telle que définie dans l'une quelconque des revendications 1 à 6, avec un ARN codant pour des protéines, dont une séquence partielle présente une similitude avec la séquence déterminante cible de ladite molécule d'acide nucléique fonctionnelle.

12. Procédé pour accroître l'efficacité de la synthèse protéique selon la revendication 11, dans lequel :
l'étape (a) comprend la transfection, dans une cellule, de la molécule d'acide nucléique fonctionnelle telle que définie dans l'une quelconque des revendications 1 à 6, ou d'une molécule d'ADN telle que définie dans la revendication 8.

13. Procédé *in vitro* de production d'une protéine, comprenant l'étape consistant à accroître l'efficacité de la synthèse protéique au moyen d'un procédé tel que défini dans les revendications 11 ou 12.

14. Molécule d'acide nucléique fonctionnelle telle que définie dans l'une quelconque des revendications 1 à 6, molécule d'ADN telle que définie dans la revendication 8, ou vecteur d'expression tel que défini dans la revendication 9, pour une utilisation en médecine.

15. Molécule d'acide nucléique fonctionnelle telle que définie dans l'une quelconque des revendications 1 à 6, molécule d'ADN telle que définie dans la revendication 8, ou vecteur d'expression tel que défini dans la revendication 9, pour une utilisation dans le traitement d'une haplo-insuffisance ou d'une maladie causée par une diminution quantitative d'une protéine.
